(19) Europäisches Patentamt
European Patent Office
Office européen des brevets

(11) EP 1 658 081 B1

(12) **EUROPEAN PATENT SPECIFICATION**

(45) Date of publication and mention of the grant of the patent:
**24.10.2007 Bulletin 2007/43**

(21) Application number: **04761485.4**

(22) Date of filing: **27.08.2004**

(51) Int Cl.:
*A61K 31/519* (2006.01) *A61K 31/5377* (2006.01)
*A61K 31/541* (2006.01) *A61K 45/06* (2006.01)
*C07D 475/04* (2006.01) *C07D 475/08* (2006.01)
*C07D 475/00* (2006.01) *A61P 37/00* (2006.01)
*A61P 37/02* (2006.01) *A61P 37/06* (2006.01)
*A61P 9/00* (2006.01) *A61P 25/00* (2006.01)
*A61P 35/00* (2006.01)

(86) International application number:
**PCT/BE2004/000124**

(87) International publication number:
**WO 2005/021003 (10.03.2005 Gazette 2005/10)**

(54) **IMMUNOSUPPRESSIVE EFFECTS OF PTERIDINE DERIVATIVES**

IMMUNOSUPPRESSIVE WIRKUNGEN VON PTERIDINDERIVATIVEN

EFFETS IMMUNOSUPPRESSIFS DE DERIVES DE PTERIDINE

(84) Designated Contracting States:
**AT BE BG CH CY CZ DE DK EE ES FI FR GB GR HU IE IT LI LU MC NL PL PT RO SE SI SK TR**

(30) Priority: **29.08.2003 US 651604**
**22.04.2004 GB 0408955**

(43) Date of publication of application:
**24.05.2006 Bulletin 2006/21**

(73) Proprietor: **4 AZA IP NV**
**3000 Leuven (BE)**

(72) Inventors:
- **WAER, Mark, Jozef, Albert**
**B-3001 Heverlee (BE)**
- **HERDEWIJN, Piet, André, Maurits, Maria**
**B-3111 Rotselaar/Wezemaal (BE)**
- **PFLEIDERER, Wolfgang, Eugen**
**D-78464 Konstanz (DE)**
- **MARCHAND, Arnaud, Didier, Marie**
**B-3001 Heverlee (BE)**
- **DE JONGHE, Steven, Cesar, Alfons**
**B-1030 Brussel (BE)**

(74) Representative: **Bird, Ariane**
**Bird Goën & Co**
**Klein Dalenstraat 42A**
**3020 Winksele (BE)**

(56) References cited:

| | |
|---|---|
| **EP-A- 0 134 922** | **EP-A- 0 185 259** |
| **EP-A- 1 479 682** | **WO-A-00/39129** |
| **WO-A-01/21619** | **WO-A-02/32507** |
| **WO-A-03/062240** | **WO-A-20/05025574** |
| **DD-A1- 267 495** | **DE-A1- 1 921 308** |
| **DE-A1- 4 009 941** | **GB-A- 785 353** |
| **US-A- 2 940 972** | **US-A- 3 081 230** |
| **US-A- 3 159 628** | **US-A- 3 859 287** |

- **MATTER HANS ET AL: "Structural requirements for inhibition of the neuronal nitric oxide synthase (NOS-I): 3D-QSAR analysis of 4-oxo- and 4-amino-pteridine-based inhibitors" JOURNAL OF MEDICINAL CHEMISTRY, vol. 45, no. 14, 4 July 2002 (2002-07-04), pages 2923-2941, XP002313348 ISSN: 0022-2623**
- **DATABASE CA [Online] CHEMICAL ABSTRACTS SERVICE, COLUMBUS, OHIO, US; KALDRIKYAN, M. A. ET AL: "Pteridine derivatives. I. Synthesis of some substituted 6,7-diarylpteridines" XP002313350 retrieved from STN Database accession no. 1976:560035 & ARMYANSKII KHIMICHESKII ZHURNAL , 29(4), 337-41 CODEN: AYKZAN; ISSN: 0515-9628, 1976,**
- **YAO, QIZINENG ET AL: "Pteridines. Part CXIII. Protection of pteridines" HELVETICA CHIMICA ACTA , 86(1), 1-12 CODEN: HCACAV; ISSN: 0018-019X, 2003, XP008041327**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- NICOLAUS B J R: "Symbiotic Approach to Drug Design" DECISION MAKING IN DRUG RESEARCH, XX, XX, 1983, pages 173-186, XP002197412
- ISRAEL, MERVYN ET AL: "Pyrimidine derivatives. VII. Some condensed derivatives of 2,4,5-triamino-6-methylthiopyrimidine" JOURNAL OF PHARMACEUTICAL SCIENCES , 54(11), 1626-32 CODEN: JPMSAE; ISSN: 0022-3549, 1965, XP008045079
- DATABASE BEILSTEIN 29 November 1988 (1988-11-29), XP002324247
- ELLIOTT R D ET AL: "Synthesis of N-10-methyl-4-thiofolic acid and related compounds." JOURNAL OF MEDICINAL CHEMISTRY. MAY 1975, vol. 18, no. 5, May 1975 (1975-05), pages 492-496, XP002324245 ISSN: 0022-2623
- FROEHLICH LOTHAR G ET AL: "Inhibition of neuronal nitric oxide synthase by 4-amino pteridine derivatives: Structure-activity relationship of antagonists of (6R)-5,6,7,8-tetrahydrobiopterin cofactor" JOURNAL OF MEDICINAL CHEMISTRY, vol. 42, no. 20, 7 October 1999 (1999-10-07), pages 4108-4121, XP002324246 ISSN: 0022-2623
- SPICKETT R G W ET AL: "THE SYNTHESIS OF COMPOUNDS WITH POTENTIAL ANTI-FOLIC ACID ACTIVITY. PART I. 7-AMINO- AND 7-HYDROXY-PTERIDINES" JOURNAL OF THE CHEMICAL SOCIETY, CHEMICAL SOCIETY. LETCHWORTH, GB, 1954, pages 2887-2891, XP008045137 ISSN: 0368-1769

## Description

The invention relates to a class of novel pteridines as specified in claim 1. The invention further relates to pharmaceutical compositions including these pteridines especially for the prevention and/or the treatment of pathologic conditions such as immune and auto-immune disorders.

The invention further relates to combined pharmaceutical preparations comprising one or more pteridines and one or more known immuno-suppressant drugs or immunomodulator drugs, antihistamines and antiallergic drugs.

This invention also relates to the use of an effective amount of a specific pteridine optionally combined with one or more known immunosuppressant drugs or immunomodulator drugs, antihistamines or antiallergic drugs, for the manufacture of a medicament for the prevention or treatment of ankylosing spondylitis, Sjögren's syndrome or asthma.

BACKGROUND OF THE INVENTION

Several 2,4-diaminopteridine derivatives being substituted in the 6-position and/or the 7-position of the pteridine ring (according to standard atom numbering for said ring) are known in the art, e.g. from various sources of literature including Swiss Patent No. 231,852; British Patent No. 763,044; United States Patents No. 2,512,572; No. 2,581,889; No. 2,665,275; No. 2,667,486; No. 2,940,972; No. 3,081,230 and No. 5,047,405. Some of these substituted 2,4-diaminopteridine derivatives were disclosed in relationship with various medical uses, such as bacterial growth inhibitors, antineoplastic agents, anti-schisto-somiasis activity, coronary dilating activity, diuretic and hypotensive activity, and anti-amnesic activity. In particular, U.S. Patent No. 2,940,972 and EP-A-362,645 disclose very specific 2,4-diaminopteridine derivatives being substituted by piperidinyl, morpholinyl or pyrrolidinyl in the 7-position of the pteridine ring.

Specific 2-aminopteridine derivatives wherein the 4-position of the pteridine ring is substituted with an alkoxy group and the 6-position is also substituted are also known in the art, although without any medical utility. For instance, United States Patent No. 2,740,784 discloses such derivatives wherein the 6-substituent is an acetal; J. Chem. Soc. (1957) 2146-2158 discloses 2-dimethylamino-4-ethoxy-6-phenylpteridine as a compound with a melting point of 200°C; Arm. Khim. J. discloses 2-amino-4-ethoxy-6,7-diphenylpteridine; Helv. Chem. Acta (1992) 75:2317-2326 discloses 2-amino-4-pentoxy-6-methylthio-pteridine. Furthermore, International Patent Application published as WO 01/19825 discloses 2-phenylamino and 2-phenylsulfoxide pteridines wherein the 7-position is substituted with an amide as being useful inhibitors of cell cycle regulatory kinases. 2-amino-4-morpholino-6-phenylpteridines optionally substituted on the phenyl ring with chloro, p-methoxy or 3,4-dimethoxy are known to be useful for the treatment of allergic diseases (WO 00/39129) and auto-immune diseases (WO 01/21619).

Nevertheless, there still is a need in the art for specific and highly therapeutically active compounds, such as drugs for treating immune and autoimmune disorders. In particular, there is a need in the art to provide immunosuppressive compounds which are active in a minor dose in order to replace existing drugs having significant side effects and to decrease treatment costs.

Currently used immunosuppressive drugs include antiproliferative agents, such as methotrexate (a 2,4-diaminopteridine derivative disclosed by U.S. Patent No. 2,512,572), azathioprine, and cyclophosphamide. Since these drugs affect mitosis and cell division, they have severe toxic effects on normal cells with high turn-over rate such as bone marrow cells and the gastrointestinal tract lining. Accordingly, marrow depression and liver damage are common side effects of these antiproliferative drugs.

Anti-inflammatory compounds used to induce immunosuppression include adrenocortical steroids such as dexamethasone and prednisolone. The common side effects observed with the use of these compounds are frequent infections, abnormal metabolism, hypertension, and diabetes.

Other immunosuppressive compounds currently used to inhibit lymphocyte activation and subsequent proliferation include cyclosporine, tacrolimus and rapamycin. Cyclosporine and its relatives are among the most commonly used immunosuppressant drugs. Cyclosporine is typically used for preventing or treating organ rejection in kidney, liver, heart, pancreas, bone marrow, and heart-lung transplants, as well as for the treatment of autoimmune and inflammatory diseases such as Crohn's disease, aplastic anemia, multiple-sclerosis, myasthenia gravis, uveitis, biliary cirrhosis, etc. However, cyclosporines suffer from a small therapeutic dose window and severe toxic effects including nephrotoxicity, hepatotoxicity, hypertension, hirsutism, cancer, and neurotoxicity.

In the field of allergy, IgE is well known for inducing allergy mainly by stimulating mast cells to release histamine. Also, asthma, being characterized by inflammation of airway and bronchospasm, is mainly induced by Th2 cytokines such as IL-5, IL-10 or IL-13. Therefore there is a need in the art for compounds that efficiently inhibit the release of these Th2 cytokines.

There is also a need in the art to improve therapeutic efficiency by providing pharmaceutical compositions or combined preparations exhibiting a synergistic effect as a result of combining two or more immunosuppressant drugs or anti-histamine drugs.

Meeting these various needs in the art constitutes the main goal of the present invention.

SUMMARY OF THE INVENTION

In a first embodiment, the present invention relates to a group of novel pteridine derivatives according to claim 1. Most of these compounds belong to a group having the general formula (I):

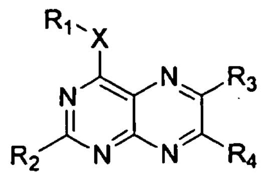

wherein X represents a group with the formula NZ and wherein:

- $R_1$ is a group selected from the group consisting of $C_{1-7}$ alkyl and arylalkyl;
- Z is hydrogen or the group NZ together with $R_1$ is morpholino;
- $R_2$ is amino;
- $R_4$ is hydrogen; and
- $R_3$ is aryl optionally substituted with one or more substituents;

and/or a pharmaceutically acceptable addition salt thereof and/or a stereoisomer thereof and/or a mono- or a di-N-oxide thereof and/or a solvate thereof and/or a dihydro- or tetrahydropteridine derivative thereof.

The above novel compounds have in common a potential specific biological activity profile and consequent usefulness in medicinal chemistry.

In a second embodiment, the present invention relates to the unexpected finding that at least one desirable biological property such as the ability to decrease the proliferation of lymphocytes, or to decrease T-cell activation, or to decrease B-cell or monocytes or macrophages activation, or to inhibit the release of certain cytokines, is a common feature which is present in the group of novel compounds defined by claim 1. As a consequence, the invention relates to pharmaceutical compositions comprising as an active principle at least one pteridine derivative as defined in claim 1.

These compounds are highly active immunosuppressive agents or anti-allergic agents which, together with one or more pharmaceutically acceptable carriers, may be formulated into pharmaceutical compositions for the prevention or treatment of pathologic conditions such as defined in claim 6.

In a further embodiment, the present invention relates to combined preparations containing at least one compound and one or more drugs such as immunosuppressant and/or immunomodulator drugs, anti-histamines, or inhibitors of agents causative of allergic conditions. In a further embodiment, the present invention relates to the prevention or treatment of the above-cited pathologic conditions by administering to the patient in need thereof an effective amount of a compound as defined in claim 1, optionally in the form of a pharmaceutical composition or combined preparation with another suitable drug.

Also disclosed are various processes and methods for making the novel pteridine derivatives of claim 1, as well as their pharmaceutically acceptable salts, N-oxides, solvates, enantiomers and dihydro- and tetrahydroderivatives.

BRIEF DESCRIPTION OF THE DRAWINGS

Figure 1 represents a scheme for the preparation of 2,4,6-trisubstituted pteridine derivatives according to one embodiment of this invention.

Figures 2 to 5 represent alternative schemes for the preparation of 2,4,6-trisubstituted pteridine derivatives according to other embodiments of this invention.

Figure 6 represents a scheme for the preparation of 2,4,6-trisubstituted pteridine derivatives according to one embodiment of this invention, wherein the substituent in the 6-position of the pteridine ring is a phenyl group substituted by a nitrogen-containing function.

Figure 7 represents a scheme for the preparation of 2,4,6-trisubstituted pteridine derivatives according to another embodiment of this invention, wherein the substituent in the 6- position of the pteridine ring is a phenyl group

substituted by an oxygen-containing function.

DEFINITIONS

Unless otherwise stated herein, the term " trisubstituted " means that three of the carbon atoms being in positions 2, 4 and 6 of the pteridine ring (according to standard atom numbering for the pteridine ring) are substituted with an atom or group other than hydrogen.

As used herein with respect to a substituting radical, and unless otherwise stated, the terms " $C_{1-7}$ alkyl" or " aliphatic saturated hydrocarbon radicals with 1 to 7 carbon atoms " means straight and branched chain saturated acyclic hydrocarbon monovalent radicals having from 1 to 7 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl; the term "$C_{1-4}$ alkyl " designates the corresponding radicals with only 1 to 4 carbon atoms, and so on.

As used herein with respect to a substituting radical, and unless otherwise stated, the term $C_{1-7}$ alkylene means the divalent hydrocarbon radical corresponding to the above defined $C_{1-7}$ alkyl, such as methylene, bis(methylene), tris (methylene), tetramethylene, hexamethylene.

As used herein with respect to a substituting radical, and unless otherwise stated, the terms $C_{3-10}$ cycloalkyl" and " cycloaliphatic saturated hydrocarbon radical with 3 to 10 carbon atoms " means a monocyclic saturated hydrocarbon monovalent radical having from 3 to 10 carbon atoms, such as for instance cyclopropyl, cyclobutyl, cyclopentyl, cyclohexyl, cycloheptyl, cyclooctyl, or a $C_{7-10}$ polycyclic saturated hydrocarbon monovalent radical having from 7 to 10 carbon atoms such as, for instance, norbornyl, fenchyl, trimethyltricycloheptyl or adamantyl.

As used herein with respect to a substituting radical, and unless otherwise stated, the term "$C_{3-10}$ cycloalkylene" means the divalent hydrocarbon radical corresponding to the above defined $C_{3-10}$ cycloalkyl.

As used herein with respect to a substituting radical, and unless otherwise stated, the terms " aryl " and "aromatic substituent " are interchangeable and designate any mono- or polyaromatic monovalent hydrocarbon radical having from 6 up to 30 carbon atoms such as phenyl, naphthyl, anthracenyl, adamantyl, phenantracyl, fluoranthenyl, chrysenyl, pyrenyl, biphenylyl, terphenyl, picenyl, including spiro hydrocarbon radicals and fused benzo - $C_{5-8}$ cycloalkyl radicals (the latter being as defined above) such as, for instance, indanyl, 1,2,3,4-tetrahydronaphtalenyl, fluorenyl

As used herein with respect to a substituting radical, and unless otherwise stated, the term " homo-cyclic" means a mono- or polycyclic, saturated or mono-unsaturated or polyunsaturated hydrocarbon radical having from 4 up to 15 carbon atoms but including no heteroatom in the said ring.

As used herein with respect to a substituting radical, and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in a 3 to 10 membered ring (and optionally one or more heteroatoms attached to one or more carbon atoms of said ring, for instance in the form of a carbonyl or thiocarbonyl group) and/or to one or more heteroatoms of said ring, for instance in the form of a sulfone, sulfoxide, N-oxide, phosphate, phosphonate or selenium oxide, each said heteroatom being independently selected from the group consisting of nitrogen, oxygen, sulfur, selenium and phosphorus, including benzo-fused heterocyclic radicals, such as but not limited to oxabicycloheptyl, azabenzimidazolyl, azacycloheptyl, azacyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thio-urazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl,cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl, thietanyl, diazabicyclo-octyl, diazetyl, diaziridinonyl, diaziridinethionyl, chromanyl, chromanonyl, thiochromanyl, thiochromanonyl, thiochromenyl, benzofuranyl, benzisothiazolyl, benzocarbazolyl, benzochromonyl, benziso-alloxazinyl, benzocoumarinyl, thiocoumarinyl, phenometoxazinyl, phenoparoxazinyl, phentriazinyl, thiodiazinyl, thiodiazolyl, indoxyl, thio-indoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido, benzylsultimyl,

benzylsultamyl, including all possible isomeric forms thereof, wherein each carbon atom of the said ring may be substituted with a substituent selected from the group consisting of halogen, nitro, $C_{1-7}$ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercapto-alkylamino, heterocyclic amino, hydrazino, alkylhydrazino, phenyl-hydrazino, sulfonyl, sulfonamido and halogen), $C_{3-7}$ alkenyl, $C_{2-7}$ alkynyl, halo $C_{1-7}$ alkyl, $C_{3-10}$ cycloalkyl, aryl, arylalkyl, alkylaryl, alkylacyl, arylacyl, hydroxyl, amino, $C_{1-7}$ alkylamino, cycloalkylamino, alkenylamino, cyclo-alkenylamino, alkynylamino, arylamino, arylalkylamino, hydroxyalkylamino, mercaptoalkylamino, hetero-cyclic amino, hydrazino, alkylhydrazino, phenylhydrazino, sulfhydryl, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the 3 to 10 membered ring, heterocyclic radicals may be sub-divided into heteroaromatic (or " heteroaryl") radicals and non-aromatic heterocyclic radicals; when a heteroatom of the said non-aromatic heterocyclic radical is nitrogen, the latter may be substituted with a substituent selected from the group consisting of $C_{1-7}$ alkyl, $C_{3-10}$ cycloalkyl, aryl, arylalkyl and alkylaryl.

As used herein with respect to a substituting radical, and unless otherwise stated, the terms " $C_{1-7}$ alkoxy ", "$C_{3-10}$ cycloalkoxy ", " aryloxy", "arylalkyloxy ", " oxyheterocyclic ", "thio $C_{1-7}$ alkyl", " thio $C_{3-10}$ cycloalkyl ", " arylthio ", " arylalkylthio " and " thioheterocyclic" refer to substituents wherein a $C_{1-7}$ alkyl radical, respectively a $C_{3-10}$ cycloalkyl, aryl, arylalkyl or heterocyclic radical (each of them such as defined herein), are attached to an oxygen atom or a sulfur atom through a single bond, such as but not limited to methoxy, ethoxy, propoxy, butoxy, thioethyl, thiomethyl, phenyloxy, benzyloxy, mercaptobenzyl, cresoxy.

As used herein with respect to a substituting atom, and unless otherwise stated, the term halogen means any atom selected from the group consisting of fluorine, chlorine, bromine and iodine.

As used herein with respect to a substituting radical, and unless otherwise stated, the term " halo $C_{1-7}$ alkyl " means a $C_{1-7}$ alkyl radical (such as above defined) in which one or more hydrogen atoms are independently replaced by one or more halogens (preferably fluorine, chlorine or bromine), such as difluoromethyl, trifluoromethyl, trifluoroethyl, octafluoropentyl, dodecafluoroheptyl, dichloromethyl; the term "halo $C_{1-4}$ alkyl " designate the corresponding radical with only 1 to 4 carbon atoms, and so on.

As used herein with respect to a substituting radical, and unless otherwise stated, the terms "$C_{2-7}$ alkenyl " and " aliphatic unsaturated hydrocarbon radical with 2 to 7 carbon atoms " are interchangeable and designate a straight and branched acyclic hydrocarbon monovalent radical having one or more ethylenical unsaturations and having from 2 to 7 carbon atoms such as, for example, vinyl, 2-propenyl, 3-butenyl, 2-butenyl, 2-pentenyl, 3-pentenyl, 3-methyl-2-butenyl, 3-hexenyl, 2-hexenyl, 2-heptenyl, butadienyl, pentadienyl, hexadienyl, heptadienyl, heptatrienyl, including all possible isomers thereof; the term " $C_{3-7}$ alkenyl " designate the corresponding radical with only 3 to 7 carbon atoms, and so on.

As used herein with respect to a substituting radical, and unless otherwise stated, the terms "$C_{3-10}$ cycloalkenyl" and " cycloaliphatic unsaturated hydrocarbon radical with 3 to 10 carbon atoms " are interchangeable and mean a monocyclic mono- or polyunsaturated hydrocarbon monovalent radical having from 3 to 8 carbon atoms, such as for instance cyclopropenyl, cyclobutenyl, cyclopentenyl, cyclopentadienyl, cyclohexenyl, cyclohexadienyl, cycloheptenyl, cycloheptadienyl, cyclohepta-trienyl, cyclooctenyl, cyclooctadienyl and the like, or a $C_{7-10}$ polycyclic mono- or polyunsaturated hydrocarbon mono-valent radical having from 7 to 10 carbon atoms such as dicyclopentadienyl, fenchenyl (including all isomers thereof, such as $\alpha$-pinolenyl), bicyclo[2.2.1]hept-2-enyl, bicyclo[2.2.1]hepta-2,5-dienyl, cyclo-fenchenyl.

As used herein with respect to a substituting radical, and unless otherwise stated, the term "$C_{2-7}$ alkynyl " defines straight and branched chain hydrocarbon radicals containing one or more triple bonds and having from 2 to 20 carbon atoms such as, for example, acetylenyl, 2-propynyl, 3-butynyl, 2-butynyl, 2-pentynyl, 3-pentynyl, 3-methyl-2-butynyl, 3-hexynyl, 2-hexynyl and all possible isomers thereof.

As used herein with respect to a substituting radical, and unless otherwise stated, the terms " arylalkyl " and "heterocyclic-substituted alkyl" refer to an aliphatic saturated hydrocarbon monovalent radical, preferably a $C_{1-7}$ alkyl or a $C_{3-10}$ cycloalkyl such as defined above, onto which an aryl radical or respectively a heterocyclic radical (such as defined above) is already bonded, such as benzyl, pyridylmethyl, pyridylethyl, 2-(2-pyridyl)isopropyl, oxazolylbutyl, 2-thienylmethyl and 2-furylmethyl.

As used herein with respect to a substituting radical, and unless otherwise stated, the term " alkylaryl " and "alkyl-substituted heterocyclic" refer to an aryl radical or respectively a heterocyclic radical (such as defined above) onto which is (are) already bonded one or more aliphatic saturated hydrocarbon monovalent radicals, preferably $C_{1-7}$ alkyl radicals or $C_{3-10}$ cycloalkyl radicals as defined above such as o-toluyl, m-toluyl, p-toluyl, mesityl and 2,4,6-trimethylphenyl.

As used herein with respect to a substituting radical, and unless otherwise stated, the terms " alkylamino ", "cycloalkylamino ", "alkenyl-amino", " cycloalkenylamino " , " arylamino ", "arylalkylamino", "heterocyclic amino " ,

" hydroxyalkylamino ", "mercaptoalkylamino " and " alkynylamino" mean that respectively one (thus monosubstituted amino) or even two (thus disubstituted amino) $C_{1-7}$ alkyl, $C_{3-10}$ cycloalkyl, $C_{2-7}$ alkenyl, $C_{3-10}$ cycloalkenyl, aryl, arylalkyl, heterocyclic, mono- or polyhydroxy $C_{1-7}$ alkyl, mono- or polymercapto $C_{1-7}$ alkyl or $C_{2-7}$ alkynyl radical(s) (each of them as defined herein, respectively) is/are attached to a nitrogen atom through a single bond or, in the case of heterocyclic, include a nitrogen atom, such as anilino, benzylamino, methylamino, dimethylamino, ethylamino, diethylamino, isopropylamino, propenylamino, n-butylamino, ter-butylamino, dibutylamino, morpholino-alkylamino, morpholinyl, piperidinyl, piperazinyl, hydroxymethylamino, β-hydroxyethylamino and ethynylamino; this definition also includes mixed disubstituted amino radicals wherein the nitrogen atom is attached to two such radicals belonging to two different sub-set of radicals, e.g. an alkyl radical and an alkenyl radical, or to two different radicals within the same sub-set of radicals, e.g. methylethylamino; the term "$C_{3-7}$ alkyl-amino" designates the corresponding radical with only 3 to 7 carbon atoms in the alkyl group(s) attached to nitrogen, for instance di-isopropylamino, and so on; among disubstituted amino radicals, symetrically substituted are usually preferred and more easily accessible.

As used herein with respect to a substituting radical, and unless otherwise stated, the terms "(thio)carboxylic acid ester " , " (thio)carboxylic acid thioester " and " (thio)carboxylic acid amide" refer to radicals wherein the carboxyl or thiocarboxyl group is directly attached to the pteridine ring (e.g. in the 6- and/or 7-position) and wherein said carboxyl or thiocarboxyl group is bonded to the hydrocarbonyl residue of an alcohol, a thiol, a polyol, a phenol, a thiophenol, a primary or secondary amine, a polyamine, an amino-alcohol or ammonia, the said hydrocarbonyl residue being selected from the group consisting of alkyl, alkenyl, alkynyl, cycloalkyl, cycloalkenyl, aryl, arylalkyl, alkylaryl, alkylamino, cycloalkylamino, alkenylamino, cycloalkenylamino, arylamino, arylalkylamino, heterocyclic amino, hydroxyalkylamino, mercapto-alkylamino or alkynylamino (such as above defined, respectively).

As used herein with respect to a substituting radical, and unless otherwise stated, the term " amino-acid " refers to a radical derived from a molecule having the chemical formula $H_2N$-CHR-COOH, wherein R is the side group of atoms characterizing the amino-acid type; said molecule may be one of the 20 naturally-occurring amino-acids or any similar non naturally-occurring amino-acid.

As used herein and unless otherwise stated, the term " stereoisomer " refers to all possible different isomeric as well as conformational forms which the compounds of formula (I) or (II) may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a pteridine derivative of this invention with a suitable inorganic solvent (e.g. hydrates) or organic solvent, such as alcohols, ketones, esters.

As used herein and unless otherwise stated, the terms " dihydro-pteridine derivative " and " tetrahydropteridine derivative " refer to the hydrogenation products of the pteridine derivatives having formula (I), i.e. derivatives wherein two hydrogen atoms are present in positions 5 and 6, or 7 and 8, of the pteridine ring, or wherein four hydrogen atoms are present in positions 5, 6, 7 and 8 of the said ring; such hydrogenated derivatives are easily accessible from the pteridine derivatives using hydrogenation methods well known in the art.

## DETAILED DESCRIPTION OF THE INVENTION

A main object of the invention is to provide a pharmaceutical composition having high immunosuppressive activity. Thus, the present invention relates in particular to the medical applications of a group of pteridine derivatives, their pharmaceutically acceptable salts, N-oxides, solvates, dihydro- and tetrahydroderivatives and enantiomers, possessing unexpectedly desirable pharmaceutical properties, in particular which are highly active immunosuppressive agents, and as such are useful in the treatment of certain inflammatory diseases.

Surprisingly, the compounds of the present invention show a broader therapeutic spectrum profile than merely immunosuppressive activity, as is evidenced by the results obtained in the diversity of test procedures disclosed hereinbelow. A further advantageous feature of the compounds of the present invention resides in their excellent oral activity.

When a mixture of enantiomers of a pteridine derivative according to the invention is obtained during synthesis, the said mixture may be separated by means and methods standard in the art, e.g. liquid chromatography using one or more suitable chiral stationary phases. The latter include, for example, polysaccharides, in particular cellulose or amylose derivatives. Commercially available polysaccharide-based chiral stationary phases suitable for this purpose are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide-based chiral stationary phases are hydrocarbons such

as hexane and the like, optionally admixed with an alcohol such as ethanol, isopropanol. The above mixture of enantiomers may alternatively be separated by forming diastereoisomers, followed by separation of the diastereoisomers, e.g. by differential crystallization or chromatography. The resolving agent may be cleaved from the separated diastereoisomers, e.g. by treatment with acids or bases, in order to generate the pure enantiomers of the compounds of the invention.

Some preferred pteridine derivatives according to the invention are more specifically illustrated in the following examples and defined in the following claims.

Also disclosed are processes and methods for making the novel pteridine derivatives. As a general rule, the preparation of these compounds is based on the principle that, starting from a suitable pteridine precursor, each of the substituents $XR_1$, $R_2$, $R_3$ and $R_4$ may be introduced separately without adversely influencing the presence of one or more substituents already introduced at other positions on the pteridine ring or the capacity to introduce further substituents later on.

Methods of manufacture have been developed by the present inventors which may be used alternatively to, or may be combined with, the methods of synthesis already known in the art of pteridine derivatives (depending upon the targeted final compound). The synthesis of mono- and di-*N*-oxides of the pteridine derivatives of this invention can easily be achieved by treating the said derivatives with an oxidizing agent such as, but not limited to, hydrogen peroxide (e.g. in the presence of acetic acid) or a peracid such as chloroperbenzoic acid. Dihydro- and tetrahydropteridine derivatives of this invention can easily be obtained by catalytic hydrogenation of the corresponding pteridine derivatives, e.g. by placing the latter in a hydrogen atmosphere in the presence of platinum oxide or platinum. The methods for making the pteridine derivatives of the present invention will now be explained in more details by reference to the appended figures 1 to 7 wherein, unless otherwise stated hereinafter, each of the substituting groups or atoms X, Z, $R_1$, $R_2$, $R_3$ and $R_4$ is as defined in formula (I) of the summary of the invention and, more specifically, may correspond to any of the individual meanings disclosed above. The same manufacturing methods may also be applied, if need be, while starting from pteridine derivatives which are already known in the art. In the description of the reaction steps involved in each figure, reference is made to the use of certain catalysts and/or certain types of solvents. It should be understood that each catalyst mentioned should be used in a catalytic amount well known to the skilled person with respect to the type of reaction involved. Solvents that may be used in the following reaction steps include various kinds of organic solvents such as protic solvents, polar aprotic solvents and non-polar solvents as well as aqueous solvents which are inert under the relevant reaction conditions. More specific examples include aromatic hydrocarbons, chlorinated hydrocarbons, ethers, aliphatic hydrocarbons, alcohols, esters, ketones, amides, water or mixtures thereof, as well as supercritical solvents such as carbon dioxide (while performing the reaction under supercritical conditions). The suitable reaction temperature and pressure conditions applicable to each kind of reaction step will not be detailed herein but do not depart from the relevant conditions already known to the skilled person with respect to the type of reaction involved and the type of solvent used (in particular its boiling point).

Figure 1 represents a scheme for the preparation of 2,4,6-trisubstituted pteridines with various $R_2$ and $R_3$ substituents in the 2- and 6- positions of the pteridine ring, respectively. In the first step (a), a chloropyrimidine 1, wherein $R_2$ may be *inter alia* amino, alkylamino, arylamino, alkoxy, aryloxy, mercaptoalkyl, or mercaptoaryl, is reacted with an appropriate nucleophile $R_1XH$, the said nucleophile being selected from the group consisting of alcohols (e.g. methanol, ethanol, isopropanol or benzylalcohol), thiols, primary amines and secondary amines wherein $R_1$ may be *inter alia* alkyl, cycloalkyl, aryl, alkylaryl, heteroaryl or alkylheteroaryl. Introduction of a nitroso group into the pyrimidine intermediate 2 occurs in step (b) under acidic aqueous conditions in the presence of sodium nitrite $NaNO_2$. Reduction of the nitroso functionality of the pyrimidine intermediate 3 into a free amino group in intermediate 4 is then effected in step (c) by means of reducing agents (such as $Na_2S_2O_4$ or $(NH_4)_2S$) in water, or catalytically ($Pt/H_2$) in the presence of a protic solvent. In step (d), ring closure is performed by treating the diaminopyrimidine 4 with glyoxal in order to form a pteridine ring. In step (e), the nitrogen atom at position 8 of the pteridine ring of compound 5 is oxidized, e.g. using $H_2O_2$ under acidic conditions. In step (f), a chlorine atom is regioselectively introduced on the 6 position of the pteridine ring of compound 6 by treatment with a carboxylic acid choride such as acetyl chloride under acidic conditions. Then in step (g) the 6-chlorosubstituted pteridine 7 is reacted with a boronic acid having the general formula $R_3B(OH)_2$, wherein $R_3$ may be alkyl, cycloalkyl, aryl or heteroaryl, under basic conditions (such as in the presence of an aqueous alcaline solution) and a palladium based catalyst, thus yielding the desired derivative 8 of the present invention.

Figure 2 represents a scheme for the preparation of 2,4,6- or 2,4,7-trisubstituted (reference) or 2,4,6,7-tetrasubstituted (reference) pteridine derivatives with various $R_1$, $R_2$, $R_3$ and/or $R_4$ substituents. In step (a), the thiol function of 2-mercapto-4,6-diaminopyrimidine is alkylated, preferably methylated by reaction with methyl iodide in the presence of a solvent such as ethanol, in order to yield 2-thiomethyl-4,6-diaminopyrimidine. Introduction of a nitroso group in the 5-position of the pyrimidine ring is then achieved in step (b) by using sodium nitrite under aqueous acidic conditions. In step (c), the methylthio group in the 2-position is exchanged for a group $R_2$ by reaction with an appropriate nucleophile, wherein $R_2$ is as defined above and preferably is primary or secondary amino, $C_{1-7}$ alkoxy, aryloxy, $C_{3-10}$ cycloalkoxy, heteroaryloxy, mercapto $C_{1-7}$alkyl, mercaptoaryl, mercapto $C_{3-10}$cycloalkyl or mercapto-heteroaryl. Reduction of the nitroso group is then achieved in step (d) either catalytically ($Pt/H_2$) in the presence of a protic solvent or chemically

using sodium dithionite or ammonium sulfide in the presence of water. Then in step (e), the resulting 2-$R_2$-substituted-4,5,6-triaminopyrimidine is condensed, under acidic conditions in the presence of a solvent such as methanol, with an $\alpha$-ketoaldoxime bearing the group $R_3$, wherein $R_3$ may be $C_{1-7}$ alkyl, $C_{3-10}$ cycloalkyl, aryl or heteroaryl, into a 2,6-substituted-4-aminopteridine derivative. Alternatively, the corresponding 2,7-substituted-4-aminopteridine derivative can be obtained in step (f) by reacting the 2-$R_2$-Substituted-4,5,6-triaminopyrimidine with a monosubstituted glyoxal bearing a group $R_4$, wherein $R_4$ may be inter alia $C_{1-7}$ alkyl, $C_{3-10}$ cycloalkyl, aryl or heteroaryl. Alternatively, a 2-$R_2$-substituted 4-amino-6,7-disubstituted pteridine derivative can be obtained in step (g) by reacting the 2-$R_2$-substituted 4,5,6-triaminopyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions. In step (h), acidic or basic hydrolysis of the amino group at position 4 of the pteridine ring is performed and results in the corresponding 4-oxopteridine derivative. In step (i), the hydroxyl group of the tautomeric form of the latter is activated by nucleophilic displacement, e.g. by preparing the 4-[(1 ,2,4)-triazolyl] pteridine derivative. Finally in a first part of step (j), a nucleophilic displacement is performed by mixing the said 4-triazolylpteridine derivative with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl yielding the desired final pteridine derivatives.

Figure 3 represents a scheme for the preparation of 2,4,6- or 2,4,7-trisubstituted (reference) or 2,4,6,7-tetrasubstituted (reference) pteridine derivatives with various substituents on the pteridine ring, starting from the 2-thiomethyl-5-nitroso-4,6-diaminopyrimidine obtained after step (b) of the scheme shown in figure 2. Reduction of the nitroso group is achieved in step (a) either catalytically ($Pt/H_2$) in the presence of a protic solvent or chemically using sodium dithionite or ammonium sulfide in the presence of water. Then in step (b), 2-thiomethyl-4,5,6-triaminopyrimidine is condensed, under acidic conditions in the presence of a solvent such as methanol, with an $\alpha$-ketoaldoxime bearing the group $R_3$, wherein $R_3$ may be *inter alia* $C_{1-7}$ alkyl, $C_{3-10}$ cycloalkyl, aryl or heteroaryl, thus regioselectively yielding a 2-thiomethyl-4-amino-6-$R_3$-substituted-pteridine derivative. Alternatively, the corresponding 2-thiomethyl-4-amino-7-$R_4$-substituted pteridine is obtained in step (c) by reacting 2-thiomethyl-4,5,6-triaminopyrimidine with a monosubstituted glyoxal bearing a group $R_4$, wherein $R_4$ may be *inter alia* $C_{1-7}$ alkyl, $C_{3-10}$ cycloalkyl, aryl or heteroaryl. Alternatively, the corresponding 2-thiomethyl-4-amino-6-$R_3$-7-$R_4$-substituted pteridine is obtained in step (d) by reacting 2-thiomethyl-4,5,6-triamino-pyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions. In step (e), the methylthio group in the 2-position is oxidized to the corresponding sulfone by using oxidizing agents such as chloroperoxybenzoic acid in chloroform or hydrogen peroxide in acetic acid. The methylsulfonyl group is easily exchanged in step (f) by reaction with a nucleophile, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl. In step (g), acidic or basic hydrolysis of the amino group at position 4 of the pteridine ring is performed and results in the corresponding 4-oxopteridine derivative. In step (h), the hydroxyl group of the tautomeric form of the latter is activated by nucleophilic displacement, e.g. by preparing the 4-[(1,2,4)-triazolyl] pteridine derivative. In the last step (i), a nucleophilic displacement is performed by mixing the said 4-triazolylpteridine derivative with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl.

Figure 4 represents a scheme for the synthesis of unsymmetrical 2,4,6-trisubstituted and 2,4,7-trisubstituted (reference), as well as 2,4,6,7-tetrasubstituted (reference), pteridine derivatives with various $R_1$, $R_2$, $R_3$ and/or $R_4$ substituents in the 2-, 4-, 6- and/or 7-positions of the pteridine ring, respectively. In step (a), a 2-$R_2$-substituted-4,5,6-triamino-pyrimidine is condensed with an $\alpha$-keto-aldoxime bearing a radical $R_3$, wherein $R_3$ may be *inter alia* selected from the group consisting of $C_{1-7}$ alkyl, $C_{3-10}$ cycloalkyl, aryl and heteroaryl, in a protic solvent such as methanol under acidic conditions yielding regioselectively a 4-amino-pteridine bearing a $R_2$-substituent in position 2 and a $R_3$ substituent in position 6 of the pteridine ring. Alternatively, a 2-$R_2$-substituted-4-amino-7-$R_4$-substituted pteridine derivative can be obtained in step (b) by reacting a 2-$R_2$-substituted-4,5,6-triamino-pyrimidine with a monosubstituted glyoxal bearing the group $R_4$, wherein $R_4$ may be *inter alia* selected from the group consisting of $C_{1-7}$ alkyl, $C_{3-10}$ cycloalkyl, aryl and heteroaryl, under neutral or basic conditions. Alternatively, a 2-$R_2$-substituted-4-amino-6,7-di-substituted pteridine derivative can be obtained in step (c) by reacting a 2-$R_2$-substituted-4,5,6-triamino-pyrimidine with a disubstituted glyoxal bearing the groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl or heteroaryl, under neutral or basic conditions. In step (d), acidic or basic hydrolysis of the amino group at position 4 of the pteridine ring is performed and results in the corresponding 4-oxo-pteridine derivative. In step (e), the hydroxyl group of the tautomeric form of the latter is activated for a nucleophilic displacement reaction, e.g. by preparing the 4-[(1,2,4)-triazolyl]pteridine derivative. In the last step (f), the 4-triazolylpteridine derivative is reacted with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl,

mercapto $C_{3-10}$ cycloalkyl, or mercapto-heteroaryl.

Figure 5 represents a scheme for the synthesis of symmetrical 2,4,6-trisubstituted and 2,4,7-trisubstituted (reference) as well as 2,4,6,7-tetrasubstituted (reference) pteridine derivatives with various $R_1$, $R_2$, $R_3$ and/or $R_4$ substituents in the 2-, 4-, 6- and/or 7-positions of the pteridine ring. In step (a), a nitroso group is introduced on position 5 of the pyrimidine ring of a 2-$R_2$-substituted 4-oxo-6-aminopyrimidine by using sodium nitrite under aqueous acidic conditions. Reduction of the nitroso group in step (b) is achieved either catalytically ($Pt/H_2$) in the presence of a protic solvent, or chemically using sodium dithionite or ammonium sulfide in water. Then in a next step (c), condensing the resulting 2-$R_2$-substituted 4-oxo-5,6-diamino-pyrimidine with an $\alpha$-ketoaldoxime bearing a radical $R_3$, wherein $R_3$ may be *inter alia* $C_{1-7}$ alkyl, $C_{3-10}$ cycloalkyl, aryl or heteroaryl, in a protic solvent such as methanol under acidic conditions regioselectively yields a 4-oxopteridine bearing a $R_2$ substituent in position 2 and a $R_3$ substituent in position 6 of the pteridine ring. Alternatively, a 2-$R_2$-substituted 4-oxo-7-$R_4$-substituted pteridine derivative can be obtained in step (d) by reacting the 2-$R_2$-substituted 4-oxo-5,6-diaminopyrimidine with a monosubstituted glyoxal bearing the group $R_4$, wherein $R_4$ may be *inter alia* $C_{1-7}$ alkyl, $C_{3-10}$ cycloalkyl, aryl or heteroaryl, under neutral or basic conditions. Alternatively, a 2-$R_2$-substituted-4-oxo-6,7-disubstituted pteridine derivative can be obtained in step (e) by reacting the 2-$R_2$-substituted 4-oxo-5,6-diamino-pyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions. Activation of the (tautomeric) hydroxyl substituent in position 4 of the pteridine ring for a nucleophilic displacement reaction occurs in step (f) by preparing the corresponding 4-[(1,2,4)-triazolyl] pteridine derivative, e.g. using $POCl_3$ or 4-chlorophenyl phosphorodichloridate and 1,2,4-triazole in pyridine as solvent. When $R_2$ is an amino group, protection of $R_2$ may further be necessary before carrying out this reaction. The amino group can be protected for instance by an acetyl group, which can be hydrolysed back to the amino group in a next step. Nucleophilic substitution is performed in step (g) by mixing the triazolyl pteridine derivative with a nucleophile $R_1XH$, (such as for example such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl) at room temperature in a polar aprotic solvent such as 1,4-dioxane.

Figure 6 represents a scheme for the preparation of 2,4,6-trisubstituted pteridine derivatives, wherein the substituent in the 6- position of the pteridine ring is a phenyl group substituted by a nitrogen-containing function. In step (a), the chlorine at position 4 of the pyrimidine ring is displaced by an appropriate nucleophile, having the general formula $R_1XH$ (such as, but not limited to, a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl). Introduction of the nitroso group at position 5 of the pyrimidine ring is achieved by treatment with sodium nitrite under aqueous acidic conditions. Reduction of the nitroso group in step (c) is achieved either catalytically ($Pt/H_2$) in the presence of a protic solvent, or chemically using sodium dithionite or ammonium sulfide in water. In step (d), the 2-$R_2$-4-$XR_1$-substituted-5,6-triamino-pyrimidine analogue is condensed with an acetamidophenylglyoxalmonoxime in a protic solvent such as methanol under acidic conditions to yield regioselectively a pteridine analogue bearing an acetamidophenyl group at position 6 of the pteridine ring. Acidic deprotection of the acetyl group leads to the formation of the free amino group. This amino group can be transformed into amides (by reaction with carboxylic acids or acid chlorides) or into sulfonamides (by reaction with sulfonyl chlorides) by reaction in a polar aprotic solvent (e.g. pyridine, dimethylformamide or dichloromethane) and optionally further in the presence of a base (e.g. triethylamine).

Figure 7 represents a scheme for the preparation of 2,4,6-trisubstituted pteridine derivatives, wherein the substituent in the 6- position of the pteridine ring is a phenyl group substituted by an oxygen-containing function. In step (a) a 2-$R_2$-4-$XR_1$-substituted-5,6-triaminopyrimidine, which may be obtained for instance after step (c) described in figure 6, is condensed with an hydroxyphenyl-glyoxalmonoxime in a protic solvent such as methanol under acidic conditions to yield regioselectively a pteridine analogue bearing an hydroxyphenyl group at position 6 of the pteridine ring. The free hydroxyl group can be alkylated in a polar protic solvent (e.g. dimethylformamide) using a base (such as potassium carbonate, cesium carbonate or sodium hydride) and an appropriate alkylhalide or arylalkylhalide.

When applicable, and depending upon the specific substituents being present, the novel pteridine derivatives may be in the form of a pharmaceutically acceptable salt. The latter include any therapeutically active non-toxic addition salt which compounds are able to form with a salt-forming agent. Such addition salts may conveniently be obtained by treating the pteridine derivatives of the invention with an appropriate salt-forming acid or base. For instance, pteridine derivatives having basic properties may be converted into the corresponding therapeutically active, non-toxic acid addition salt form by treating the free base form with a suitable amount of an appropiate acid following conventional procedures. Examples of such appropriate salt-forming acids include, for instance, inorganic acids resulting in forming salts such as hydrohalides (e.g.hydrochloride and hydrobromide), sulfate, nitrate, phosphate, diphosphate, carbonate, bicarbonate; and organic monocarboxylic or dicarboxylic acids resulting in forming salts such as, for example, acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, 2-hydroxybenzoate, 4-amino-2-hydroxybenzoate, benzene-sulfonate, p-toluenesulfonate, salicylate, p-aminosalicylate, pamoate, bitartrate, camphorsulfonate,

edetate, 1,2-ethanedisulfonate, fumarate, glucoheptonate, gluconate, glutamate, hexylresorcinate, hydroxynaphtoate, hydroxyethanesulfonate, mandelate, methylsulfate, pantothenate, stearate, as well as salts derived from ethanedioic, propanedioic, butanedioic, (Z)-2-butenedioic, (E)2-butenedioic, 2-hydroxybutanedioic, 2,3-dihydroxy-butanedioic, 2-hydroxy-1,2,3-propanetricarboxylic and cyclohexanesulfamic acids.

Pteridine derivatives having acidic properties may be converted in a similar manner into the corresponding therapeutically active, non-toxic base addition salt form. Examples of appropriate salt-forming bases include, for instance, inorganic bases like metallic hydroxides such as those of alkali and alkaline-earth metals like calcium, lithium, magnesium, potassium and sodium, or zinc, resulting in the corresponding metal salt; organic bases such as ammonia, alkylamines, benzathine, hydrabamine, arginine, lysine, N,N'-dibenzylethylenediamine, chloroprocaine, choline, diethanolamine, ethylene-diamine, N-methylglucamine, procaine.

Reaction conditions for treating the pteridine derivatives of this invention with an appropriate salt-forming acid or base are similar to standard conditions involving the same acid or base but different organic compounds with basic or acidic properties, respectively. Preferably, in view of its use in a pharmaceutical composition or in the manufacture of medicament for treating specific diseases, the pharmaceutically acceptable salt will be designed, i.e. the salt-forming acid or base will be selected so as to impart greater water-solubility, lower toxicity, greater stability and/or slower dissolution rate to the pteridine derivative of this invention.

The present invention further provides the use of a pteridine derivative according to claim 1 or a pharmaceutically acceptable salt or a solvate thereof, for the manufacture of a medicament for the prevention or treatment of a pathologic condition as defined in claim 6

The pathologic conditions and disorders concerned by the said use are detailed hereinbelow.

The invention further relates to a pharmaceutical composition comprising:

(a) one or more pteridine, and
(b) one or more pharmaceutically acceptable carriers.

In a third embodiment, this invention provides combinations, preferably synergistic combinations, of one or more pteridine with one or more biologically-active drugs being preferably selected from the group consisting of immunosuppressant and/or immunomodulator drugs, anti-histamines, and inhibitors of allergy-causative agents (anti-allergic drugs). As is conventional in the art, the evaluation of a synergistic effect in a drug combination may be made by analyzing the quantification of the interactions between individual drugs, using the median effect principle described by Chou et al. in Adv. Enzyme Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1c, 2c), which is needed to produce a given effect. The said first and second drug have synergistic or additive or antagonistic effects depending upon $CI < 1$, $CI = 1$, or $CI > 1$, respectively. As will be explained in more detail herein-below, this principle may be applied to a number of desirable effects such as an activity against immunosuppression or immunomodulation, or an activity against allergy.

For instance the present invention relates to a pharmaceutical composition or combined preparation having synergistic effects against immuno-suppression or immunomodulation and containing:

(a) one or more immunosuppressant and/or immunomodulator drugs, and
(b) at least one pteridine derivative according to claim 1, and
(c) optionally one or more pharmaceutical excipients or pharmaceutically acceptable carriers, for simultaneous, separate or sequential use in the treatment or prevention of ankylosing spondilytis or Sjögren syndrome.

Suitable immunosuppressant drugs for inclusion in the synergistic compositions or combined preparations of this invention belong to a well known therapeutic class. They are preferably selected from the group consisting of cyclosporin A, substituted xanthines (e.g. methylxanthines such as pentoxyfylline), daltroban, sirolimus, tacrolimus, rapamycin (and derivatives thereof such as defined below), leflunomide (or its main active metabolite A771726, or analogs thereof called malononitrilamides), mycophenolic acid and salts thereof (including the sodium salt marketed

under the trade name Mofetil®), adrenocortical steroids, azathioprine, brequinar, gusperimus, 6-mercaptopurine, mizoribine, chloroquine, hydroxychloroquine and monoclonal antibodies with immunosuppressive properties (e.g. etanercept, infliximab or kineret). Adrenocortical steroids within the meaning of this invention mainly include glucocorticoids such as ciprocinonide, desoxycorticisterone, fludrocortisone, flumoxonide, hydrocortisone, naflocort, procinonide, timobesone, tipredane, dexamethasone, methylprednisolone, methotrexate, prednisone, prednisolone, triamcinolone and pharmaceutically acceptable salts thereof. Rapamycin derivatives as referred herein include O-alkylated derivatives, particularly 9-deoxorapamycins, 26-dihydrorapamycins, 40-O-substituted rapamycins and 28,40-O,O-disubstituted rapamycins (as disclosed in U.S. Patent No. 5,665,772) such as 40-O-(2-hydroxy) ethyl rapamycin - also known as SDZ-RAD -, pegylated rapamycin (as disclosed in U.S. Patent No. 5,780,462), ethers of 7-desmethylrapamycin (as disclosed in U.S. Patent No. 6,440,991) and polyethylene glycol esters of SDZ-RAD (as disclosed in U.S. Patent No. 6,331,547).

Suitable immunomodulator drugs for inclusion into the synergistic immunomodulating pharmaceutical compositions or combined preparations of this invention are preferably selected from the group consisting of acemannan, amiprilose, bucillamine, dimepranol, ditiocarb sodium, imiquimod, Inosine Pranobex, interferon-β, interferon-γ, lentinan, levamisole, lisophylline, pidotimod, romurtide, platonin, procodazole, propagermanium, thymomodulin, thymopentin and ubenimex.

Synergistic activity of the pharmaceutical compositions or combined preparations of this invention against immunosuppression or immuno-modulation may be readily determined by means of one or more lymphocyte activation tests. Usually activation is measured via lymphocyte proliferation. Inhibition of proliferation thus always means immunosuppression under the experimental conditions applied. There exist different stimuli for lymphocyte activation, in particular:

a) co-culture of lymphocytes of different species (mixed lymphocyte reaction, hereinafter referred as MLR) in a so-called mixed lymphocyte culture test: lymphocytes expressing different minor and major antigens of the HLA-DR type (= alloantigens) activate each other non-specifically;
b) a CD3 assay wherein there is an activation of the T-lymphocytes via an exogenously added antibody (OKT3). This antibody reacts against a CD3 molecule located on the lymphocyte membrane which has a co-stimulatory function. Interaction between OKT3 and CD3 results in T-cell activation which proceeds via the $Ca^{2+}$/calmodulin/calcineurin system and can be inhibited e.g. by cyclosporin A (hereinafter referred as CyA);
c) a CD28 assay wherein specific activation of the T-lymphocyte proceeds via an exogenously added antibody against a CD28 molecule which is also located on the lymphocyte membrane and delivers strong co-stimulatory signals. This activation is $Ca^{2+}$-independent and thus cannot be inhibited by CyA.

Determination of the immunosuppressing or immunomodulating activity of the pteridine derivatives of this invention, as well as synergistic combinations comprising them, is preferably based on the determination of one or more, preferably at least three lymphocyte activation *in vitro* tests, more preferably including at least one of the MLR test, CD3 assay and CD28 assay referred above. Preferably the lymphocyte activation *in vitro* tests used include at least two assays for two different clusters of differentiation preferably belonging to the same general type of such clusters and more preferably belonging to type I transmembrane proteins. Optionally the determination of the immuno-suppressing or immunomodulating activity may be performed on the basis of other lymphocyte activation *in vitro* tests, for instance by performing a TNF-α assay or an IL-1 assay or an IL-6 assay or an IL-10 assay or an IL-12 assay or an assay for a cluster of differentiation belonging to a further general type of such clusters and more preferably belonging to type II transmembrane proteins such as CD69, CD 71 or CD1 34.

The synergistic effect may be evaluated by the median effect analysis method described herein-before. Such tests may for instance, according to standard practice in the art, involve the use of equiment, such as flow cytometer, being able to separate and sort a number of cell subcategories at the end of the analysis, before these purified batches can be analysed further.

The pharmaceutical composition or combined preparation with synergistic activity against immunosuppression or immunomodulation according to this invention may contain the pteridine derivative over a broad content range depending on the contemplated use and the expected effect of the preparation. Generally, the pteridine derivative content of the combined preparation is within the range of 0.1 to 99.9% by weight, preferably from 1 to 99% by weight, more preferably from 5 to 95% by weight.

The pharmaceutical compositions and combined preparations according to this invention may be administered orally or in any other suitable fashion. Oral administration is preferred and the preparation may have the form of a tablet, aqueous dispersion, dispersable powder or granule, emulsion, hard or soft capsule, syrup, elixir or gel. The dosing forms may be prepared using any method known in the art for manufacturing these pharmaceutical compositions and may comprise as additives sweeteners, flavoring agents, coloring agents, preservatives and the like. Carrier materials and excipients are detailed hereinbelow and may include, *inter alia,* calcium carbonate, sodium carbonate, lactose, calcium phosphate or sodium phosphate; granulating and disintegrating agents, binding agents. The pharmaceutical composition

or combined preparation of this invention may be included in a gelatin capsule mixed with any inert solid diluent or carrier material, or has the form of a soft gelatin capsule, in which the ingredient is mixed with a water or oil medium. Aqueous dispersions may comprise the biologically active composition or combined preparation in combination with a suspending agent, dispersing agent or wetting agent. Oil dispersions may comprise suspending agents such as a vegetable oil. Rectal administration is also applicable, for instance in the form of suppositories or gels. Injection (e.g. intramuscularly or intraperiteneously) is also applicable as a mode of administration, for instance in the form of injectable solutions or dispersions, depending upon the disorder to be treated and the condition of the patient.

Auto-immune disorders to be prevented or treated by the pharmaceutical compositions or combined preparations of this invention are selected from the systemic auto-immune diseases ankylosing spondilytis, and Sjögren syndrome;

Allergic conditions to be prevented or treated by the pharmaceutical compositions of this invention are selected from severe forms, namely asthma, characterized by inflammation of airways and bronchospasm. Without wishing to be bound by theory, the antiallergic effect of the compounds of the invention may be related to their suppression of certain B-cell activation pathways, which can lead to the suppression of IgE release. It may also be related to their properties of inhibiting certain Th2 cytokines, such as IL-5, IL-13 or IL-10, involved in asthma.

The term " pharmaceutically acceptable carrier or excipient " as used herein in relation to pharmaceutical compositions and combined preparations means any material or substance with which the active principle, i.e. the pteridine derivative, and optionally the immunosuppressant or immunomodulator, may be formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the pteridine derivatives of this invention, special attention will be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride), provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents. may also be prepared by micronisation, for instance in view to obtain them in the form of microspheres usually having a diameter of about 1 to 10 $\mu$m, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanyl-phosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated

and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenolpolyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2nd ed. (Hanser Verlag, Vienna, 1981) and "Encyclopaedia of Surfactants (Chemical Publishing Co., New York, 1981).

Structure-forming, thickening or gel-forming agents may be included into the pharmaceutical compositions and combined preparations of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included into the pharmaceutical compositions and combined preparations of the present invention include cellulose derivatives such as carboxymethylcellulose, cellulose acetate and the like; natural gums such as arabic gum, xanthum gum, tragacanth gum, guar gum; gelatin; silicon dioxide; synthetic polymers such as carbomers, and mixtures thereof. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite, and mixtures thereof; preservatives such as, for example, potassium sorbate, sodium benzoate, sorbic acid, propyl gallate, benzylalcohol, methyl paraben, propyl paraben; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins, and mixtures thereof.

Since, in the case of combined preparations including the pteridine derivative of this invention and an immunosuppressant or immunomodulator or antihistamine, both ingredients do not necessarly bring out their synergistic therapeutic effect directly at the same time in the patient to be treated, the said combined preparation may be in the form of a medical kit or package containing the two ingredients in separate but adjacent form. In the latter context, each ingredient may therefore be formulated in a way suitable for an administration route different from that of the other ingredient, e.g. one of them may be in the form of an oral or parenteral formulation whereas the other is in the form of an ampoule for intravenous injection or an aerosol.

The following examples are intended to illustrate the present invention.

Example 66 - synthesis of 4,5,6-triamino-2-methylmercaptopyrimidine dihydro-chloride (intermediate)

To a suspension of a 2-methylmercapto-4,5,6-triamino-pyrimidine sulfate (44.3 mmole), which may be prepared and characterised for instance as disclosed by Taylor et al. in J. Am. Chem. Soc. (1952) 74:1644-1647, in water (135 ml) at 80 °C was added dropwise a solution of barium chloride dihydrate (39.8 mmole) in water (25 ml). The suspension was stirred for 30 minutes at 80 °C. The reaction mixture was cooled down and barium sulfate was filtered off over Celite. The filtrate was evaporated in vacuo and co-evaporated with toluene yielding the title compound as a yellow powder (10.2 g, 94 % yield).

Example 67 - synthesis of 4-amino-2-methylmercapto-6-(3,4-dimethoxy-phenyl) pteridine (intermediate)

To a suspension of 4,5,6-triamino-2-methylmercaptopyrimidine dihydrochloride (7.42 mmole, 1.81 g) in methanol (20 ml) was added a solution of 3,4-dimethoxyphenylglyoxaloxime (5.94 mmole, 1.24 g) in methanol. The resulting reaction mixture was refluxed for 3 hours. The reaction mixture was neutralised with concentrated aqueous ammonia until pH 9 was reached. The resulting precipitate was filtered off and further purified by flash chromatography (silica, using an ethyl acetate / hexane mixture in a 4:6 ratio) yielding the pure title compound as a yellow powder which was characterised as follows: MS: m/z (%) 330 ($[M+H]^+$, 100), 681 ($[2M+Na]^+$, 30); UV (MeOH, nm): 292, 397.

Example 68 - synthesis of 2,4-di-(thienyl-2-methylamino)-6-(3,4-dimethoxy-phenyl) pteridine

A solution of the compound of example 67 (100 mg, 0.304 mmole) in 2-thiophenemethylamine (12 ml) was refluxed overnight. The solvents were removed in vacuo and the residue was purified first by flash chromatography (silica, gradient from 2:98 to 3:97 $CH_3OH$ /$CH_2Cl_2$) and then by preparative TLC (silica, EtOAc/hexane 1 :1) yielding the title compound as a yellow powder (85 mg, 57 %) which was characterised as follows : MS: m/z (%): 491 ($[M+H]^+$, 100); UV (MeOH, nm): 229, 296, 414.

Example 69 - synthesis of 2,5,6-triamino-4-morpholino-pyrimidine dihydro-chloride (intermediate)

In a 10 L 4-neck flask equipped with a mechanical stirrer, a thermometer and a heating mantle was placed 2,6-diamino-4-chloropyrimidine (870.5 g; 6.0 mol) in water (3190 ml). To the stirred resulting white suspension was added morpholine (1113 g; 12.8 moles) in one portion. The temperature raised from 14 to 28°C and the mixture was heated until at 75°C a clear solution was obtained and refluxed for 16 hours. After complete conversion of the starting material (controlled by HPLC analysis) the reaction mixture was cooled to room temperature and $NaNO_2$ (465 g, 6.74 mole) was added in three portions. No heat effect was observed but the product precipitated out of the mixture. During subsequent dropwise addition of acetic acid (600 g, 9.0 moles) the mixture became so thick that faster stirring and additional water (850 mL) were necessary to achieve a well mixable suspension. A temperature raise from 15 to 28°C was measured. After stirring for 2hours, HPLC analysis showed complete conversion. The mixture was then allowed to cool down to room temperature and transferred to a 20L flask. To the acid (pH 4) purple slurry was added water (3500 mL) and then an aqueous NaOH solution (165 g in 825 mL) in order to neutralise to pH 7. The suspension was cooled down to 10-12°C in an ice-water bath and $Na_2S_2O_4$ (4.68 kg, 26.9 moles) was added in portions of about 500 g at 5 minutes intervals. A temperature raise to 30°C was observed. The mixture was left stirring overnight at room temperature before being analysed (HPLC: 99.3% conversion). The suspension was filtered over filter cloth and the wet cake was washed with 2.77 L water leaving 1.5 kg of wet material which was slurried in isopropanol (10.2 L) and to the suspension was added dropwise 36% $HCl/H_2O$ (1.16L) during which the temperature raised from 15 to 30°C. Subsequent heating of the slurry to 40°C afforded an almost clear solution and crystallisation started suddenly showing an exotherm to 50°C. The suspension was cooled down to room temperature before the suspension was filtered over filter cloth. The wet cake was again slurried in isopropanol (5.0 L), again filtered over filter cloth and dried at 40°C in vacuo (200 mbar) for several days. According to this procedure was obtained 1.2 kg (yield 54.1 %) of 2,5,6-triamino-4-morpholino-pyrimidine dihydrochloride with a purity above 99.9% and characterised as follows: UV (MeOH, nm): 225, 254.

Example 70 - synthesis of 4-acetamidophenylglyoxalmonoxime (intermediate)

$SeO_2$ (34.18 g, 0.308 mole) and 4-acetamidoacetophenone (49.62 g, 0.28 mole) were suspended in a mixture of dioxane (250 ml) and water (10 ml) and the solution was heated to 100°C for 16 hours. The hot solution was filtered on a paper filter and the filtrate was evaporated to dryness. The oily residue was then partitioned between $CHCl_3$ (400 ml) and a saturated solution of sodium hydrogen-carbonate (200 ml). A precipitation occurs in the aqueous layer. The organic phase was collected and the aqueous layer was filtered over a fritted glass. The solid (4-acetamidoglyoxaldehyde) was washed with water and kept for the next step. The aqueous layer was extracted several times with $CHCl_3$ and all

fractions were collected and evaporated to dryness. The oily residue and the precipitate were put together and suspended in a mixture of water (240 ml) and MeOH (60 ml). Then acetonoxime (20.46 g, 0.28 mole) was added and the pH was adjusted to a range of 3 - 4 with 1 N HCl. The solution was heated to 70 °C for 1 hour, then cooled to 0 °C and the resulting crystals collected. After washing with cold water, then diethylether, drying in a vacuum dessicator over $P_2O_5$ afforded 33.6 g of the desired compound (yield 58%) as yellowish crystals with a purity of 95%, which were characterised as follows: MS: m/z (%): 207 ($[M+H]^+$, 100); UV (MeOH, nm): 241.6, 278.3.

Example 71 - synthesis of 3-acetamidophenylglyoxalmonoxime (intermediate)

$SeO_2$ (36.62 g, 0.33 mole) and 3-acetamidoacetophenone (53.16 g, 0.30 mole) were suspended in a mixture of dioxane (250 ml) and water (10 ml) and the solution was heated to 100°C for 16 hours. The hot solution was filtered on a paper filter and the filtrate was evaporated to dryness. The oily residue was then suspended in a mixture of water (240 ml) and MeOH (60 ml) and acetonoxime (21.93 g, 0.30 mol) was added and the pH was adjusted to a range of 3 - 4 with 6 N HCl. The solution was heated to 70 °C for 2 hours and after cooling, the brownish precipitate was filtered and re-crystallised from toluene to afford 45.0 g of the desired compound (yield 72%) with a purity of 98%, which was characterised as follows: MS: m/z (%): 207 ($[M+H]^+$, 100), 229 ($[M+Na]^+$,80); UV (MeOH. nm): 239.2.

Example 72 - synthesis of 2-amino-4-morpholino-6-(4-acetanilide) pteridine

2,4,5-triamino-6-morpholinopyrimidine dihydrochloride (14.1 g, 50 mmole) was refluxed in MeOH (300 ml) and the compound of example 70 (11.33 g, 55 mmole) in MeOH (150 ml) was added. The mixture was refluxed for 3 hours and after cooling, the yellowish precipitate was filtered, washed with water, methanol, ether and dried at 110°C for 3 hours to afford 15.7 g of the desired product (yield 86%) which, after re-crystallisation from DMF/$H_2O$, was obtained with a purity of 97% and characterised as follows: MS: m/z (%): 366 ($[M+H]^+$, 100), 753 ($[2M+Na]^+$ ,15); UV (MeOH, nm): 213, 307, 408.

Example 73 - synthesis of 2-amino-4-morpholino-6-(3-acetanilide) pteridine

Repeating the procedure of example 72 but starting from the compound of example 71, the title pteridine derivative (16.1 g) was obtained with a yield of 88% and a purity of 97%, and characterised as follows: MS: m/z (%): 366 ($[M+H]^+$, 100), 753 ($[2M+Na]^+$ ,80); UV (MeOH, nm): 220, 294, 402.

Example 74 - synthesis of 2-amino-4-morpholino-6-(4-aminophenyl) pteridine

The crude compound of example 72 (20 mmole) was refluxed in a mixture of MeOH/HCl 6N - 1/1 (400 ml) for 2 hours and cooled in an ice bath. The remaining solid was filtered and the pH was adjusted to 10 with NaOH (10N). The orange precipitate was filtered, washed with water, ethanol, ether and dried in a vacuum dessicator over $P_2O_5$ to afford 4.7 g (yield: 73%) of the desired compound as an orange solid with a purity of 98%, which was characterised as follows: MS: m/z (%): 324 ($[M+H]^+$, 100); UV (MeOH, nm): 216, 316, 422; $^1H$ NMR (200 MHz, in DMSO-d6, ppm): 3.78 (br s, 4H); 4.33 (br s, 4H); 5.54 (br s, 2H); 6.63 (br s, 2H); 6.67 (d, 2H, J = 8.4 Hz); 7.75 (d, 2H, J = 8.4 Hz); 9.15 (s, 1H).

Example 75 - synthesis of 2-amino-4-morpholino-6-(3-aminophenyl) pteridine

Repeating the procedure of example 74 but starting from the compound of example 73, the title pteridine derivative (4.88 g) was obtained with a yield of 74% and a purity of 99.34%, and characterised as follows: MS: m/z (%): 324 ($[M+H]^+$, 100); UV (MeOH, nm): 218, 292, 403.

Examples 76 to 82 - coupling reaction between a 2-amino-4-morpholino-6-(4-amino-phenyl) pteridine and a carboxylic acid chloride, sulfonyl chloride or carbamoyl chloride

The compound of example 74 (162 mg, 0.5 mmole) was suspended in dioxane (10 ml) and triethylamine was added (84 µl, 0.6 mmole). Then a suitable carboxylic acid chloride or sulfonyl chloride or carbamoyl chloride (0.55 mmole) was added and the mixture was stirred at room temperature until completion of the reaction. After evaporation to dryness, the crude resulting product was purified by flash chromatography on silica gel using a gradient of MeOH (1-5%) in dichloromethane. Yields varied from 50 to 80%. Using this procedure, the following pteridine derivatives were prepared:

- 2-amino-4-morpholino-6-(4-benzoylaminophenyl) pteridine (example 76), purity 98.94%, characterised as follows:

MS: m/z (%): 428 ([M+H]$^+$, 100), 877 ([2M+Na]$^+$, 40); UV (MeOH, nm): 313, 408;

- 2-amino-4-morpholino-6-(4-phenoxyacetylaminophenyl) pteridine (example 77), purity 97.18%, characterised as follows: MS: m/z (%): 458 ([M+H]$^+$, 100); UV (MeOH, nm): 305, 407;
- 2-amino-4-morpholino-6-(4-propionylaminophenyl) pteridine (example 78), purity 98.86%, characterised as follows: MS: m/z (%): 380 ([M+H]$^+$, 100), 781 ([2M+Na]$^+$, 20); UV (MeOH, nm): 213, 307, 408;
- 2-amino-4-morpholino-6-(4-furoylaminophenyl) pteridine (example 79), purity 93.96%, characterised as follows: MS: m/z (%): 418 ([M+H]$^+$, 100); UV (MeOH, nm): 213, 316, 408;
- 2-amino-4-morpholino-6-(4-cyclohexanoylaminophenyl) pteridine (example 80), purity 96.91%, characterised as follows: MS: m/z (%): 434 ([M+H]$^+$, 100), 889 ([2M+Na]$^+$, 10); UV (MeOH, nm): 308, 408;
- 2-amino-4-morpholino-6-[4-(4-chlorobenzoyl)aminophenyl] pteridine (example 81), purity 96.48%, characterised as follows: MS: m/z (%): 462 ([M+H]$^+$, 100); UV (MeOH, nm): 313.9, 407; and
- 2-amino-4-morpholino-6-(4-benzyloxyacetylaminophenyl) pteridine (example 82), purity 98.45%, characterised as follows: MS: m/z (%): 472 ([M+H]$^+$, 100), 942 ([2M+H]$^+$, 5), 965 ([2M+Na]$^+$, 10); UV (MeOH, nm): 307, 407.

Examples 83 to 97 - coupling reaction between a 2-amino-4-morpholino-6-aminophenyl pteridine and a carboxylic acid chloride, sulfonyl chloride or carbamoyl chloride

The compound of example 74 or example 75 (162 mg, 0.5 mmole) was suspended in pyridine (10 ml). Then a suitable carboxylic acid chloride or sulfonyl chloride or carbamoyl chloride (0.55 mmole) was added and the mixture was stirred at room temperature until completion of the reaction. After evaporation to dryness, the crude resulting product was purified by flash chromatography on silica gel using a gradient of MeOH (1-5%) in dichloromethane. Yields varied from 20 to 80%. Using this procedure, the following pteridine derivatives were prepared:

- 2-amino-4-morpholino-6-(4-isonicotinoylaminophenyl) pteridine (example 83) was characterised as follows: MS: m/z (%): 429 ([M+H]$^+$, 100), 879 ([2M+Na]$^+$, 5); UV (MeOH, nm): 213, 313, 407; purity 95.01 %;
- 2-amino-4-morpholino-6-(4-naphtoylaminophenyl) pteridine (example 84) was characterised as follows: MS: m/z (%): 478 ([M+H]$^+$ , 100), 977 ([2M+Na]$^+$, 5); UV (MeOH, nm): 213, 313, 407; purity 95.01 %;
- 2-amino-4-morpholino-6-(4-methylsulfonylaminophenyl) pteridine (example 85) was characterised as follows: MS: m/z (%): 502 ([M+H]$^+$, 100); UV (MeOH, nm): 301, 422; purity 96.81 %;
- 2-amino-4-morpholino-6-(4-ethylsuccinylaminophenyl) pteridine (example 86) was characterised as follows: MS: m/z (%): 452 ([M+H]$^+$, 100), 925 ([2M+Na]$^+$, 15); UV (MeOH, nm): 213, 308, 408; purity: 98.98%;
- 2-amino-4-morpholino-6-[4-(4-methylbenzoate)aminophenyl) pteridine (example 87) was characterised as follows: MS: m/z (%): 486 ([M+H]$^+$, 100); UV (MeOH, nm): 236, 315, 407; purity 99.57%;
- 2-amino-4-morpholino-6-(3-benzoylaminophenyl) pteridine (example 88) was characterised as follows: MS: m/z (%): 428 ([M+H]$^+$, 100); UV (MeOH, nm): 216, 293, 402; purity: 96.46%;
- 2-amino-4-morpholino-6-(3-benzensulfonylaminophenyl) pteridine (example 89) was characterised as follows: MS: m/z (%): 464 ([M+H]$^+$, 100); UV (MeOH, nm): 216, 295, 402; purity: 97.49%;
- 2-amino-4-morpholino-6-(3-phenoxyacetylaminophenyl) pteridine (example 90) was characterised as follows: MS: m/z (%): 458 ([M+H]$^+$, 100); UV (MeOH, nm): 219, 294, 402; purity 98.96%;
- 2-amino-4-morpholino-6-(3-isonicotinoylaminophenyl) pteridine (example 91) was characterised as follows: MS: m/z (%): 429 ([M+H]$^+$, 100); UV (MeOH, nm): 216, 294, 402; purity 93.92%;
- 2-amino-4-morpholino-6-(3-cyclohexanoylaminophenyl) pteridine (example 92) was characterised as follows: MS: m/z (%): 434 ([M+H]$^+$, 100); UV (MeOH, nm): 222, 245, 294, 402; purity 99.50%;
- 2-amino-4-morpholino-6-[3-(4-methylbenzoate)aminophenyl] pteridine (example 93) was characterised as follows: MS: m/z (%): 486 ([M+H]$^+$, 100); UV (MeOH, nm): 218, 238, 294, 402; purity 97.44%;
- 2-amino-4-morpholino-6-(3-ethylsuccinylaminophenyl) pteridine (example 94) was characterised as follows: MS: m/z (%): 452 ([M+H]$^+$, 100); UV (MeOH, nm): 222, 245, 294, 402; purity 94.16%;
- 2-amino-4-morpholino-6-(3-ethylmalonylaminophenyl) pteridine (example 95) was characterised as follows: MS: m/z (%): 438 ([M+H]$^+$, 100); UV (MeOH, nm): 220, 244, 294, 402; purity 90.89%;
- 2-amino-4-morpholino-6-(3-benzyloxyacetylaminophenyl) pteridine (example 96) was characterised as follows: MS: m/z (%): 472 ([M+H]$^+$, 100); UV (MeOH, nm): 216, 243, 294, 402; purity 99.70%; and
- 2-amino-4-morpholino-6-(3-ethylsulfonylaminophenyl)pteridine (example 97) was characterised as follows: MS: m/z (%): 4716 ([M+H]$^+$, 100); UV (MeOH, nm): 218, 295, 402; purity 92.54%.

Examples 98 to 101 - coupling reaction between 2-amino-4-morpholino-6-(3-aminophenyl) pteridine and various carboxylic acids

The compound of example 75 (323 mg, 1 mmole), a carboxylic acid (1.1 mmole) and DIEA (2 mmole) were

suspended in dry DMF (10 ml) and benzotriazol-1-yloxy-tris(dimethylamino)phosphonium hexafluorophosphate) was added (1.1 mmole). The mixture was stirred at room temperature until completion of the reaction and then diluted with water. The aqueous layer was extracted with chloroform and the organic layer was evaporated to dryness. The crude resulting product was purified on silica gel using a gradient of MeOH (1-5%) in dichloromethane. Yields varied from 20 to 50%. The following compounds were made according to this procedure:

- 2-amino-4-morpholino-6-[3-Boc-(L)-phenylalanine-aminophenyl] pteridine (example 98) was characterised as follows: MS: m/z (%): 1141 ([2M+H]$^+$, 10), 571 ([M+H]$^+$, 100), 515 ([M-tBu+H]$^+$, 50). 471 ([M-Boc+H]$^+$, 10); UV (MeOH. nm): 213, 245, 294, 402; purity 96.72%;
- 2-amino-4-morpholino-6-[3-Boc-(D)-phenylalanine-aminophenyl] pteridine (example 99) was characterised as follows: MS: m/z (%): 571 ([M+H]$^+$, 100), 515 ([M-tBu+H]$^+$, 50), 471 ([M-Boc+H]$^+$, 10); UV (MeOH, nm): 213, 245, 294, 402; purity: 95.30%;
- 2-amino-4-morpholino-6-[3-Boc-(L)-tryptophane-aminophenyl] pteridine (example 100) was characterised as follows: MS: m/z (%): 610 ([M+H]$^+$, 100), 554 ([M-tBu+H]$^+$, 50), 510 ([M-Boc+H]$^+$, 10); UV (MeOH, nm): 220, 291, 402; purity 91.74%; and
- 2-amino-4-morpholino-6-[3-Boc-(D)-tryptophane-aminophenyl] pteridine (example 101) was characterised as follows: MS: m/z (%): 610 ([M+H]$^+$, 100), 554 ([M-tBu+H]$^+$, 50), 510 ([M-Boc+H]$^+$, 10); UV (MeOH, nm): 220, 291, 402; purity 84.25%.

Example 102 - synthesis of 2-amino-4-morpholino-6-(4-hydroxyphenyl) pteridine

To a solution of 2,5,6-triamino-4-morpholino-pyrimidine dihydrochloride salt (5.05 g, 17.8 mmole) in methanol (120 ml) was added 4-hydroxyphenylglyoxalmonoxime (2.95 g, 17.8 mmole). The reaction mixture was refluxed for 3 hours and then cooled down to room temperature. The yellow precipitate was filtered off, washed with water yielding the title compound as a chromatographically pure yellow powder which was further characterised as follows: MS: m/z (%): 671 ([2M+Na]$^+$, 5), 325 ([M+H]$^+$, 100); UV (MeOH, nm): 213, 302, 411.

Examples 103 to 111 - synthesis of 2-amino-4-morpholino-6-(4-alkoxy-phenyl) pteridines

To a solution of the compound of example 102 (0.65 mmole, 210 mg) in DMF was added $K_2CO_3$ (0.78 mmole, 108 mg) and an appropriate alkyl halide (0.78 mmole). The reaction was stirred overnight at room temperature. The reaction was quenched with water and extracted with $CH_2Cl_2$. The organic phase was evaporated in vacuo and the residue purified by flash chromatography (silica, gradient from 1:99 to 3:97 $CH_3OH/CH_2Cl_2$) yielding the title compound as a yellow powder in yields ranging from 15 to 55 %. The following compounds were made using this procedure:

- 2-amino-4-morpholino-6-(4-ethoxyphenyl) pteridine (example 103) was obtained from iodoethane and characterised as follows: MS: m/z (%): 727 ([2M+Na]$^+$, 5), 353 ([M+H]$^+$, 100); UV (MeOH, nm): 211, 302, 410;
- 2-amino-4-morpholino-6-(4-benzyloxyphenyl) pteridine (example 104) was obtained from benzyl bromide and characterised as follows: MS: m/z (%): 727 ([2M+Na]$^+$, 5), 353 ([M+H]$^+$, 100); UV (MeOH, nm): 245, 302, 410;
- 2-amino-4-morpholino-6-(4-(phenethyloxy)-phenyl) pteridine (example 105) was obtained from phenylethyl bromide and characterised as follows: MS: m/z (%): 428 ([M+H]$^+$, 100); UV (MeOH, nm): 245, 303, 410;
- 2-amino-4-morpholino-6-(4-phenoxy-butyronitrile) pteridine (example 106) was obtained from 4-bromobutyronitrile and characterised as follows: MS: m/z (%): 391 ([M+H]$^+$, 100);
- 2-amino-4-morpholino-6-(4-propoxy-phenyl) pteridine (example 107) was obtained from propyl iodide and characterised as follows: MS: m/z (%): 367 ([M+H]$^+$, 100);
- 2-amino-4-morpholino-6-(4-phenoxy-butyric acid ethyl ester) pteridine (example 108) was obtained from ethyl-4-bromobutyrate and characterised as follows: MS: m/z (%): 439 ([M+H]$^+$, 100);
- 2-amino-4-morpholino-6-(4-phenoxy-acetic acid ethyl ester) pteridine (example 109) was obtained from ethyl bromoacetate and characterised as follows: MS: m/z (%): 843 ([2M+H]$^+$, 100), 411 ([M+H]$^+$, 100;
- 2-amino-4-morpholino-6-(4-(2-methoxyethoxy)-phenyl) pteridine (example 110) was obtained from 2-bromoethylmethylether and characterised as follows: MS: m/z (%): 787 ([2M+H]$^+$, 25), 383 ([M+H]$^+$, 100); and
- 2-amino-4-morpholino-6-(4-butoxy-phenyl)-pteridine (example 111) was obtained from bromobutane and characterised as follows: MS: m/z (%): 381 ([M+H]$^+$, 100).

Examples 112 to 120 - synthesis of 2-amino-4-alkylamino-6-aryl-pteridines and 2-amino-4-arylalkylamino-6-aryl-pteridines

To a suspension of 2-acetylamino-4-(1,2,4-triazolyl)-6-[3,4-(dimethoxyphenyl)]pteridine (0.5 mmole) in dioxane

(5 ml) was added the desired alkylamine or arylalkylamine (1 mmole). The suspension was stirred at room temperature for 16 hours. The solvent was evaporated *in vacuo* yielding crude 2-acetylamino-4-alkylamino-6-[3,4-(dimethoxyphenyl)] pteridine or 2-acetylamino-4-arylalkylamino-6-[3,4-(dimethoxyphenyl)] pteridine. Deprotection of the acetyl group was achieved by dissolving the crude residue in a mixture of $CH_3OH$ / 20% $K_2CO_3$ in water (1:1). The solution was stirred at room temperature for 16 hours. Evaporation of the solvents *in vacuo,* followed by purification of the residue by preparative TLC (silica, using a $CH_3OH/CH_2Cl_2$ mixture (5:95) as the eluent) afforded the desired compound as a yellow powder in yields ranging from 30 to 65 %, depending upon the starting alkylamine or arylalkylamine.

The following compounds were synthesized according to this procedure and characterized as follows:

- 2-amino-4-(diethanolamino)-6-[[3,4-(dimethoxyphenyl)]pteridine (example 112) synthesized from diethanolamine; MS: *m/z* (%): 387 ($[M+H]^+$, 100);
- 2-amino-4-(benzylamino)-6-[[3,4-(dimethoxyphenyl)]pteridine (example 113) synthesized from benzylamine; MS: *m/z* (%): 389 ($[M+H]^+$, 100);
- 2-amino-4-(phenylethylamino)-6-[[3,4-(dimethoxyphenyl)]pteridine (example 114) synthesized from phenethylamine; MS: *m/z* (%): 403 ($[M+H]^+$, 100);
- 2-amino-4-(4-methyl-piperidine)-6-[[3,4-(dimethoxyphenyl)]pteridine (example 115) synthesized from 4-methyl-piperidine; MS: *mlz* (%): 381 ($[M+H]^+$, 100);
- 2-amino-4-(2-thienylmethylamino)-6-[[3,4-(dimethoxyphenyl)lpteridine (example 116) synthesized from 2-thienylamine; MS: m/z (%): 395 ($[M+H]^+$, 100);
- 2-amino-4-thiomorpholine-6-[[3,4-(dimethoxyphenyl)]pteridine (example 118) synthesized from thiomorpholine; MS: m/z (%): 385 ($[M+H]^+$, 100);
- 2-amino-4-((*R*)-sec-butylamine)-6-[[3,4-(dimethoxyphenyl)]pteridine (example 119) synthesized from (*R*)-sec-butylamine; MS: m/z (%): 355 ($[M+H]^+$, 100); and
- 2-amino-4-((*S*)-sec-butylamine)-6-[[3,4-(dimethoxyphenyl)]pteridine (example 120) synthesized from (*S*)-sec-butylamine; MS: m/z (%): 355 ($[M+H]^+$, 100).

Example 121 - mixed Lymphocyte reaction assay

Pteridine derivatives were first dissolved (10mM) in dimethylsulfoxide (hereinafter referred as DMSO) and further diluted in culture medium before use for the following *in vitro* experiments. The commercially available culture medium consisted of RPMI-1640 + 10% foetal calf serum (FCS). Some pteridine derivatives described in the previous examples (as indicated in table 3 below) were tested in the following mixed lymphocyte reaction (MLR) assay.

Peripheral blood mononuclear cells (hereinafter referred as PBMC) were isolated from heparinized peripheral blood by density gradient centrifugation over Lymphoprep (Nycomed, Maorstua, Norway). Allogeneic PBMC or Eppstein-Barr Virus-transformed human B cells [commercially available under the trade name RPMI 1788 (ATCC name CCL156)] which strongly express B7-1 and B7-2 antigens were used as stimulator cells after irradiation with 30 Gy. MLR was performed in triplicate wells. After 5 days incubation at 37°C, 1 μCi [$^3$H]-thymidine was added to each cup. After a further 16 hours incubation, cells were harvested and counted in a ß-counter. Inhibition of proliferation by a compound (drug) described in some of the previous examples was counted using the formula:

$$\% \text{ inhibition} = \frac{(\text{cpm} + \text{drugs}) - (\text{cpm cult. med})}{(\text{cpm} - \text{drugs}) - (\text{OD cult. med})} \times 100$$

wherein cpm is the thymidine count per minute. The MLR assay is regarded by those skilled in the art as an *in vitro* analogue of the transplant rejection since it is based on the recognition of allogeneic major histocompatibility antigens on the stimulator leukocytes, by responding lymphocytes.

Table 3 below shows the $IC_{50}$ values for various pteridine derivatives of this invention in the MLR test. The $IC_{50}$ value represents the lowest concentration of said pteridine derivative (expressed in μmole/l) that resulted in a 50 % suppression of the MLR.

TABLE 3

| Example n° | MLR | Example n° | MLR | Example n° | MLR |
|---|---|---|---|---|---|
| 68 | 0.8 | 72 | 8.6 | 73 | 6.4 |

(continued)

| Example n° | MLR | Example n° | MLR | Example n° | MLR |
|---|---|---|---|---|---|
| 74 | 1.1 | 75 | 4.1 | 76 | 2.1 |
| 77 | 2.5 | 78 | 6.5 | 79 | 7.8 |
| 80 | 4.1 | 83 | 3.9 | 84 | 4.0 |
| 88 | 4.9 | 89 | 0.8 | 90 | 10.0 |
| 91 | 10.0 | 92 | 2.9 | 96 | 4.1 |
| 97 | 3.3 | 98 | 3.9 | 99 | 4.1 |
| 100 | 0.9 | 101 | 0.6 | 102 | 4.3 |
| 103 | 3.7 | 107 | 6.8 | 110 | 5.4 |
| 112 | 6.0 | 114 | 6.0 | 115 | 0.8 |
| 116 | 4.1 | 117 | 0.9 | 118 | 0.4 |
| 119 | 0.5 | 120 | 0.2 | | |

Example 122 - TNF-α assay

Peripheral blood mononuclear cells (herein referred as PBMC), in response to stimulation by lipopolysaccharide (hereinafter LPS), a gram-negative bacterial endotoxin, produce various chemokines, in particular human TNF-α. Inhibition of the activation of PBMC can therefore be measured by the level of suppression of the production of TNF-α by PBMC in response to stimulation by LPS.

Such inhibition measurement was performed as follows: PBMC were isolated from heparinized peripheral blood by density gradient centrifugation over Lymphoprep (commercially available from Nycomed, Norway). LPS was then added to the PMBC suspension in complete medium ($10^6$ cells /ml) at a final concentration of 1 μg/ml. The pteridine derivative to be tested was added at different concentrations (0.1 μM, 1 μM and 10 μM) and the cells were incubated at 37°C for 72 hours in 5% $CO_2$. The supernatants were collected, then TNF-α concentrations were measured with an anti-TNF-α antibody in a sandwich ELISA (Duo Set ELISA human TNFα, commercially available from R&D Systems, United Kingdom). The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland): a standard curve (recombinant human TNFα) was drawn and the amount (pg/ml) of each sample on the standard curve was determined.

The % suppression of human TNFα production by the pteridine derivatives of the invention (drugs) was calculated using the formula :

$$\text{\% suppression} = \frac{\text{pg/ml in drugs} - \text{pg/ml in cult. med.}}{(\text{pg/ml in cult. med.+ LPS}) - \text{pg/ml cult. med.}}$$

Table 4 below shows the $IC_{50}$ values (expressed in μM) of the tested pteridine derivatives in the TNF-α assay.

TABLE 4

| Example n° | TNF-α | Example n° | TNF-α | Example n° | TNF-α |
|---|---|---|---|---|---|
| 72 | 8.4 | 74 | 2.5 | 77 | 4.9 |
| 83 | 4.4 | 88 | 4.3 | 89 | 0.5 |
| 91 | 7.3 | 92 | 5.0 | 94 | 5.5 |
| 96 | 7.7 | 97 | 10.0 | 98 | 2.9 |
| 102 | 2.1 | 103 | 10.0 | 107 | 10.0 |

(continued)

| Example n° | TNF-α | Example n° | TNF-α | Example n° | TNF-α |
|---|---|---|---|---|---|
| 108 | 0.9 | 109 | 0.6 | 110 | 7.2 |
| 112 | 3.4 | 113 | 6.5 | 114 | 7.1 |
| 115 | 0.3 | 116 | 7.2 | 117 | 0.5 |
| 118 | 0.5 | 119 | 0.2 | 120 | 0.3 |

## Claims

**1.** A pteridine derivative selected from the group consisting of:

- 2-amino-4-morpholino-6-(4-acetanilide) pteridine,
- 2-amino-4-morpholino-6-(3-acetanilide) pteridine,
- 2-amino-4-morpholino-6-(4-aminophenyl) pteridine,
- 2-amino-4-morpholino-6-(3-aminophenyl) pteridine,
- 2-amino-4-morpholino-6-(4-benzoylaminophenyl) pteridine,
- 2-amino-4-morpholino-6-(4-phenoxyacetylaminophenyl) pteridine,
- 2-amino-4-morpholino-6-(4-propionylaminophenyl) pteridine,
- 2-amino-4-morpholino-6-(4-furoylaminophenyl) pteridine,
- 2-amino-4-morpholino-6-(4-cyclohexanoylaminophenyl) pteridine,
- 2-amino-4-morpholino-6-[4-(4-chlorobenzoyl)aminophenyl] pteridine,
- 2-amino-4-morpholino-6-(4-benzyloxyacetylaminophenyl) pteridine,
- 2-amino-4-morpholino-6-(4-isonicotinoylaminophenyl) pteridine;
- 2-amino-4-morpholino-6-(4-naphtoylaminophenyl) pteridine;
- 2-amino-4-morpholino-6-(4-methylsulfonylaminophenyl) pteridine;
- 2-amino-4-morpholino-6-(4-ethylsuccinylaminophenyl) pteridine;
- 2-amino-4-morpholino-6-[4-(4-methylbenzoate)aminophenyl) pteridine;
- 2-amino-4-morpholino-6-(3-benzoylaminophenyl) pteridine;
- 2-amino-4-morpholino-6-(3-benzensulfonylaminophenyl) pteridine,
- 2-amino-4-morpholino-6-(3-phenoxyacetylaminophenyl) pteridine;
- 2-amino-4-morpholino-6-(3-isonicotinoylaminophenyl) pteridine;
- 2-amino-4-morpholino-6-(3-cyclohexanoylaminophenyl) pteridine;
- 2-amino-4-morpholino-6-[3-(4-methylbenzoate)aminophenyl] pteridine;
- 2-amino-4-morpholino-6-(3-ethylsuccinylaminophenyl) pteridine;
- 2-amino-4-morpholino-6-(3-ethylmalonylaminophenyl) pteridine;
- 2-amino-4-morpholino-6-(3-benzyloxyacetylaminophenyl) pteridine,
- 2-amino-4-morpholino-6-(3-ethylsulfonylaminophenyl)pteridine,
- 2-amino-4-morpholino-6-[3-Boc-(L)-phenylalanine-aminophenyl] pteridine;
- 2-amino-4-morpholino-6-[3-Boc-(D)-phenylalanine-aminophenyl] pteridine;
- 2-amino-4-morpholino-6-[3-Boc-(L)-tryptophane-aminophenyl] pteridine;
- 2-amino-4-morpholino-6-[3-Boc-(D)-tryptophane-aminophenyl] pteridine,
- 2-amino-4-morpholino-6-(4-hydroxyphenyl) pteridine,
- 2,4-di-(thienyl-2-methylamino)-6-(3,4-dimethoxy-phenyl) pteridine,
- 2-amino-4-morpholino-6-(4-ethoxyphenyl) pteridine;
- 2-amino-4-morpholino-6-(4-benzyloxyphenyl) pteridine;
- 2-amino-4-morpholino-6-(4-(phenethyloxy)-phenyl) pteridine,
- 2-amino-4-morpholino-6-(4-phenoxy-butyronitrile) pteridine;
- 2-amino-4-morpholino-6-(4-propoxy-phenyl) pteridine;
- 2-amino-4-morpholino-6-(4-phenoxy-butyric acid ethyl ester) pteridine;
- 2-amino-4-morpholino-6-(4-phenoxy-acetic acid ethyl ester) pteridine;
- 2-amino-4-morpholino-6-(4-(2-methoxyethoxy)-phenyl) pteridine;
- 2-amino-4-morpholino-6-(4-butoxy-phenyl)-pteridine;
- 2-amino-4-(diethanolamino)-6-[[3,4-(dimethoxyphenyl)]pteridine;
- 2-amino-4-(benzylamino)-6-[[3,4-(dimethoxyphenyl)]pteridine;

- 2-amino-4-(phenylethylamino)-6-[[3,4-(dimethoxyphenyl)]pteridine;
- 2-amino-4-(4-methyl-piperidine)-6-[[3,4-(dimethoxyphenyl)]pteridine;
- 2-amino-4-(2-thienylmethylamino)-6-[[3,4-(dimethoxyphenyl)]pteridine;
- 2-amino-4-thiomorpholine-6-[[3,4-(dimethoxyphenyl)]pteridine;
- 2-amino-4-((R)-sec-butylamine)-6-[[3,4-(dimethoxyphenyl)]pteridine; and
- 2-amino-4-((S)-sec-butylamine)-6-[[3,4-(dimethoxyphenyl)]pteridine

**2.** A pharmaceutical composition comprising as an active principle at least one pteridine derivative according to claim 1.

**3.** A pharmaceutical composition according to claim 2, further comprising one or more biologically active drugs selected from the group consisting of immuno-suppressant and/or immunomodulator drugs, antihistamines, and anti-allergic drugs.

**4.** A pharmaceutical composition according to claim 3, wherein said biologically active drug is an immunosuppressant drug selected from the group consisting of cyclosporin A; pentoxyfylline; daltroban, sirolimus, tacrolimus; rapamycin; leflunomide; mycophenolic acid and salts thereof; azathioprine, brequinar; gusperimus; 6-mercaptopurine; mizoribine; chloroquine; hydroxychloroquine; etanercept; infliximab; and kineret.

**5.** A pharmaceutical composition according to claim 3, wherein said biologically active drug is an immunomodulator drug selected from the group consisting of acemannan, amiprilose, bucillamine, ditiocarb sodium, imiquimod, Inosine Pranobex, interferon-β, interferon-γ, lentinan, levamisole, pidotimod, romurtide, platonin, procodazole, propagermanium, thymomodulin, thymopentin and ubenimex.

**6.** Use of a pteridine derivative according to claim 1 for the manufacture of a medicament for the prevention or treatment of a disease selected from the group consisting of ankylosing spondylitis, Sjögren's syndrome, and asthma.

**Patentansprüche**

**1.** Pteridinderivat, ausgewählt aus der Gruppe, bestehend aus:

- 2-Amino-4-morpholino-6-(4-acetanilid)pteridin,
- 2-Amino-4-morpholino-6-(3-acetanilid)pteridin,
- 2-Amino-4-morpholino-6-(4-aminophenyl)pteridin,
- 2-Amino-4-morpholino-6-(3-aminophenyl)pteridin,
- 2-Amino-4-morpholino-6-(4-benzoylaminophenyl)pteridin,
- 2-Amino-4-morpholino-6-(4-phenoxyacetlylaminophenyl)pteridin,
- 2-Amino-4-morpholino-6-(4-propionylaminophenyl)pteridin
- 2-Amino-4-morpholino-6-(4furoylaminophenyl)pteridin,
- 2-Amino-4-morpholino-6-(4-cyclohexanoylaminophenyl)pteridin,
- 2-Amino-4-morpholino-6-[4-(-chlorbenzoyl)aminophenyl]pteridin,
- 2-Amino-4-morpholino-6-(4-benzyloxyacetylaminophenyl)pteridin,
- 2-Amino-4-morpholino-6-(4-isonicotinoylaminophenyl)pteridin:
- 2-Amino-4-morpholino-6-(4-naphthoylaminophenyl)pteridin;
- 2-Amino-4-morpholino-6-(4-methylsulfonylaminophenyl)pteridin;
- 2-Amino-4-morpholino-6-(4-ethylsuccinylaminophenyl)pteridin;
- 2-Amino-4-morpholino-6-[4-(4-methylbenzoat)aminophenyl)]pteridin;
- 2-Amino-4-morpholino-6-(3-benzoylaminophenyl)pteridin;
- 2-Amino-4-morpholino-6-(3-benzolsulfonylaminophenyl)pteridin,
- 2-Amino-4-morpholino-6-(3-phenoxyacetylaminophenyl)pteridin;
- 2-Amino-4-morpholino-6-(3-isonicotinoylaminophenyl)pteridin;
- 2-Amino-4-morpholino-6-(3-cyclohexanoylaminophenyl)pteridin;
- 2-Amino-4-morpholino-6-[3-(4-methylbenzoat)aminophenyl]pteridin;
- 2-Amino-4-morpholino-6-(3-ethylsuccinylaminophenyl)pteridin;
- 2-Amino-4-morpholino-6-(3-ethylmalonylaminophenyl)pteridin;
- 2-Amino-4-morpholino-6-(3-benzyloxyacetylaminophenyl)pteridin,
- 2-Amino-4-morpholino-6-(3-ethylsulfonylaminophenyl)pteridin;
- 2-Amino-4-morpholino-6-[3-Boc-(L)-phenylalanin-aminophenyl]pteridin;

- 2-Amino-4-morpholino-6-[3-Boc-(D)-phenylalanin-aminophenyl]pteridin;
- 2-Amino-4-morpholino-6-[3-Boc-(L)-tryptophan-aminophenyl]pteridin;
- 2-Amino-4-morpholino-6-[3-Boc-(D)-tryptophan-aminophenyl]pteridin,
- 2-Amino-4-morpholino-6-(4-hydroxyphenyl)pteridin,
- 2,4-Di-(thienyl-2-methylamino)-6-(3,4-dimethoxy-phenyl)pteridin,
- 2-Amino-4-morpholino-6-(4-ethoxyphenyl)pteridin;
- 2-Amino-4-morpholino-6-(4-benzyloxyphenyl)pteridin;
- 2-Amino-4-morpholino-6-(4-(phenethyloxy)-phenyl)pteridin,
- 2-Amino-4-morpholino-6-(4-phenoxy-butyronitril)pteridin;
- 2-Amino-4-morpholino-6-(4-propoxy-phenyl)pteridin;
- 2-Amino-4-morpholino-6-(4-phenoxy-buttersäureethylester)pteridin;
- 2-Amino-4-morpholino-6-(4-phenoxy-essigsäureethylester)pteridin;
- 2-Amino-4-morpholino-6-(4-(2-methoxyethoxy)-phenyl)pteridin;
- 2-Amino-4-morpholino-6-(4-butoxy-phenyl)-pteridin;
- 2-Amino-4-(diethanolamino)-6-[3,4-(dimethoxyphenyl)]pteridin;
- 2-Amino-4-(benzylamino)-6-[3,4-(dimethoxyphenyl)]pteridin;
- 2-Amino-4-(phenylethylamino)-6-[3,4-(dimethoxyphenyl)]pteridin;
- 2-Amino-4-(4-methyl-piperidin)-6-[3,4-dimethoxyphenyl)]pteridin;
- 2-Amino-4-(2-thienylmethylamino)-6-[3,4-(dimethoxyphenyl)]pteridin;
- 2-Amino-4-thiomorpholin-6-[3,4-(dimethoxyphenyl)]pteridin;
- 2-Amino-4-((R)-sek.-butylamin)-6-[3,4-(dimethoxyphenyl)]pteridin;

und

- 2-Amino-4-((S)-sek-butylamin)-6-[3,4-(dimethoxyphenyl)]pteridin.

**2.** Pharmazeutische Zusammensetzung, umfassend als ein aktives Prinzip zumindest ein Pteridinderivat nach Anspruch 1.

**3.** Pharmazeutische Zusammensetzung nach Anspruch 2, weiterhin umfassend ein oder mehrere biologisch aktive Wirkstoffe, ausgewählt aus der Gruppe, bestehend aus immunsuppressiven und/oder immunmodulatorischen Wirkstoffen, Antihistaminika und antiallergischen Wirkstoffen.

**4.** Pharmazeutische Zusammensetzung nach Anspruch 3, wobei der biologisch aktive Wirkstoff ein immunsuppressiver Wirkstoff ist, ausgewählt aus der Gruppe, bestehend aus Cyclosporin A; Pentoxyfyllin; Daltroban, Sirolimus, Tacrolimus; Rapamycin; Leflunomid; Mycophenolsäure und Salze hiervon; Azathioprin, Brequinar; Gusperimus; 6-Mercaptopurin; Mizoribin; Chloroquin; Hydroxychloroquin; Etanercept; Infliximab; und Kineret.

**5.** Pharmazeutische Zusammensetzung nach Anspruch 3, wobei der biologisch aktive Wirkstoff ein immunmodulatorischer Wirkstoff ist, ausgewählt aus der Gruppe, bestehend aus Acemannan, Amiprilose, Bucillamin, Ditiocarb-Natrium, Imiquimod, Inosin Pranobex, Interferon-β, Interferon-γ, Lentinan, Levamisol, Pidotimod, Romurtid, Platonin, Procodazol, Propagermanium, Thymomodulin, Thymopentin und Ubenimex.

**6.** Verwendung eines Pteridinderivats nach Anspruch 1 zur Herstellung eines Medikaments zur Prophylaxe oder Behandlung einer Erkrankung, ausgewählt aus der Gruppe, bestehend aus ankylosierender Spondylitis, Sjögren's Syndrom und Asthma.

## Revendications

**1.** Dérivé de ptéridine choisi dans le groupe constitué par :

- la 2-amino-4-morpholino-6-(4-acétanilide)ptéridine ;
- la 2-amino-4-morpholino-6-(3-acétanilide)ptéridine ;
- la 2-amino-4-morpholino-6-(4-aminophényl)ptéridine ;
- la 2-amino-4-morpholino-6-(3-aminophényl)ptéridine ;
- la 2-amino-4-morpholino-6-(4-benzoylaminophényl)ptéridine ;
- la 2-amino-4-morpholino-6-(4-phénoxyacétylaminophényl)ptéridine ;
- la 2-amino-4-morpholino-6-(4-propionylaminophényl)ptéridine ;
- la 2-amino-4-morpholino-6-(4-furoylaminophényl)ptéridine ;

- la 2-amino-4-morpholino-6-(4-cyclohexanoylaminophényl)ptéridine ;
- la 2-amino-4-morpholino-6-[4-(4-chlorobenzoyl)aminophényl]ptéridine ;
- la 2-amino-4-morpholino-6-(4-benzyloxyacétylaminophényl)ptéridine ;
- la 2-amino-4-morpholino-6-(4-isonicotinoylaminophényl)ptéridine ;
- la 2-amino-4-morpholino-6-(4-naphtoylaminophényl)ptéridine ;
- la 2-amino-4-morpholino-6-(4-méthylsulfonylaminophényl)ptéridine ;
- la 2-amino-4-morpholino-6-(4-éthylsuccinylaminophényl)ptéridine ;
- la 2-amino-4-morpholino-6-[4-(4-méthylbenzoate)aminophényl)ptéridine ;
- la 2-amino-4-morpholino-6-(3-benzoylaminophényl)ptéridine ;
- la 2-amino-4-morpholino-6-(3-benzénsulfonylaminophényl)ptéridine ;
- la 2-amino-4-morpholino-6-(3-phénoxyacétylaminophényl)ptéridine ;
- la 2-amino-4-morpholino-6-(3-isonicotinoylaminophényl)ptéridine ;
- la 2-amino-4-morpholino-6-(3-cyclohexanoylaminophényl)ptéridine ;
- la 2-amino-4-morpholino-6-[3-(4-méthylbenzoate)aminophényl]ptéridine ;
- la 2-amino-4-morpholino-6-(3-éthylsuccinylaminophényl)ptéridine ;
- la 2-amino-4-morpholino-6-(3-éthylmalonylaminophényl)ptéridine ;
- la 2-amino-4-morpholino-6-(3-benzyloxyacétylaminophényl)ptéridine ;
- la 2-amino-4-morpholino-6-(3-éthylsulfonylaminophényl)ptéridine ;
- la 2-amino-4-morpholino-6-[3-Boc-(L)-phénylalanine-aminophényl]-ptéridine ;
- la 2-amino-4-morpholino-6-[3-Boc-(D)-phénylalanine-aminophényl]-ptéridine ;
- la 2-amino-4-morpholino-6-[3-Boc-(L)-tryptophane-aminophényl]-ptéridine ;
- la 2-amino-4-morpholino-6-[3-Boc-(D)-tryptophane-aminophényl]-ptéridine ;
- la 2-amino-4-morpholino-6-(4-hydroxyphényl)ptéridine ;
- la 2,4-di-(thiényl-2-méthylamino)-6-(3,4-diméthoxy-phényl)ptéridine
- la 2-amino-4-morpholino-6-(4-éthoxyphényl)ptéridine ;
- la 2-amino-4-morpholino-6-(4-benzyloxyphényl)ptéridine ;
- la 2-amino-4-morpholino-6-(4-(phénéthyloxy)-phényl)ptéridine ;
- la 2-amino-4-morpholino-6-(4-phénoxy-butyronitrile)ptéridine ;
- la 2-amino-4-morpholino-6-(4-propoxy-phényl)ptéridine ;
- la 2-amino-4-morpholino-6-(éthylester d'acide 4-phénoxy-butyrique)-ptéridine;
- la 2-amino-4-morpholino-6-(éthylester d'acide 4-phénoxy-acétique)-ptéridine ;
- la 2-amino-4-morpholino-6-(4-(2-méthoxyéthoxy)-phényl)ptéridine ;
- la 2-amino-4-morpholino-6-(4-butoxy-phényl)ptéridine ;
- la 2-amino-4-(diéthanolamino)-6-[3,4-(diméthoxyphényl)]ptéridine ;
- la 2-amino-4-(benzylamino)-6-[3,4-(diméthoxyphényl)]ptéridine ;
- la 2-amino-4-(phényléthylamino)-6-[3,4-(diméthoxyphényl)]ptéridine ;
- la 2-amino-4-(4-méthyl-pipéridine)-6-[3,4-(diméthoxyphényl)]ptéridine ;
- la 2-amino-4-(2-thiénylméthylamino)-6-[3,4-(diméthoxyphényl)]ptéridine ;
- la 2-amino-4-thiomorpholine-6-[3,4-(diméthoxyphényl)]ptéridine ;
- la 2-amino-4-((*R*)-sec-butylamine)-6-[3,4-(diméthoxyphényl)]ptéridine ; et
- la 2-amino-4-((*S*)-sec-butylamine)-6-[3,4-(diméthoxyphényl)]ptéridine.

**2.** Composition pharmaceutique comprenant en tant que principe actif au moins un dérivé de ptéridine selon la revendication 1.

**3.** Composition pharmaceutique selon la revendication 2, comprenant en outre un ou plusieurs médicaments biologiquement actifs choisis dans le groupe constitué par des médicaments immunosuppresseurs et/ou immunomodulateurs, des antihistaminiques et des médicaments anti-allergiques.

**4.** Composition pharmaceutique selon la revendication 3, dans laquelle ledit médicament biologiquement actif est un médicament immunosuppresseur choisi dans le groupe constitué par la cyclosporine A ; la pentoxyfylline ; le daltroban ; le sirolimus ; le tacrolimus ; la rapamycine ; le léflunomide ; l'acide mycophénolique et des sels de celuici ; l'azathioprine ; le bréquinar ; le guspérimus ; la 6-mercaptopurine ; la mizoribine ; la chloroquine ; l'hydroxychloroquine ; l'étanercept ; l'infliximab ; et le kineret.

**5.** Composition pharmaceutique selon la revendication 3, dans laquelle ledit médicament biologiquement actif est un médicament immunomodulateur choisi dans le groupe constitué par l'acemannan, l'amiprilose, la bucillamine, le ditiocarb sodium, l'imiquimod, l'Inosine Pranobex, l'interféron-β, l'interféron-y, le lentinan, le lévamisole, le pidotimod,

le romurtide, la platonine, le procodazole, le propagermanium, la thymomoduline, la thymopentine et l'ubénimex.

6. Utilisation d'un dérivé de ptéridine selon la revendication 1 pour la fabrication d'un médicament destiné à la prévention ou au traitement d'une maladie choisie dans le groupe constitué par la spondylarthrite ankylosante, le syndrome de Sjögren et l'asthme.

## Figure 1

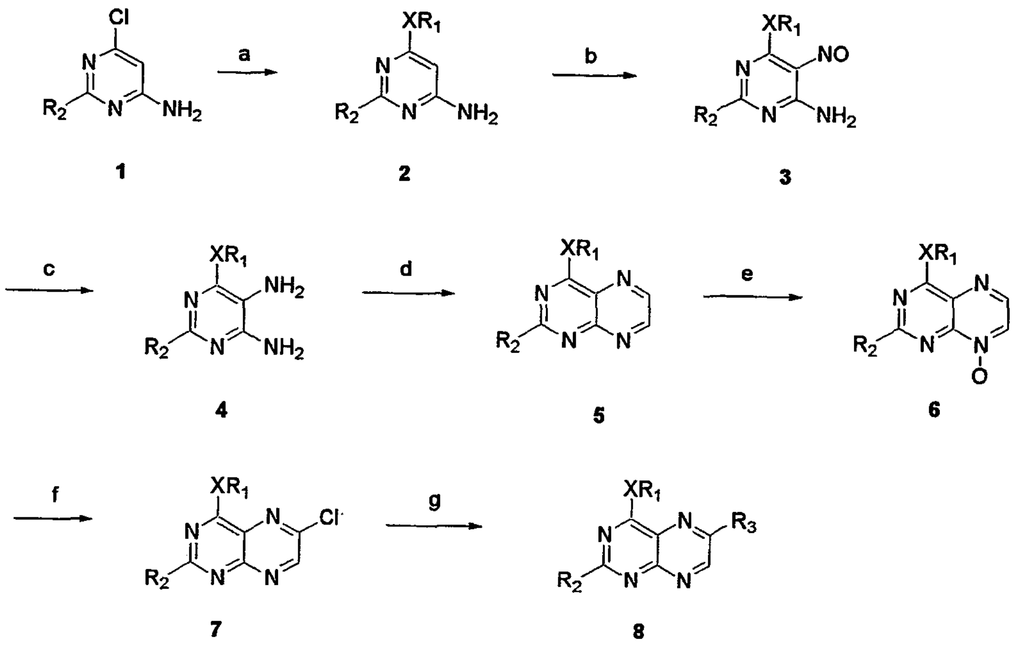


1 2 3 4 5 6 7 8

Figure 2

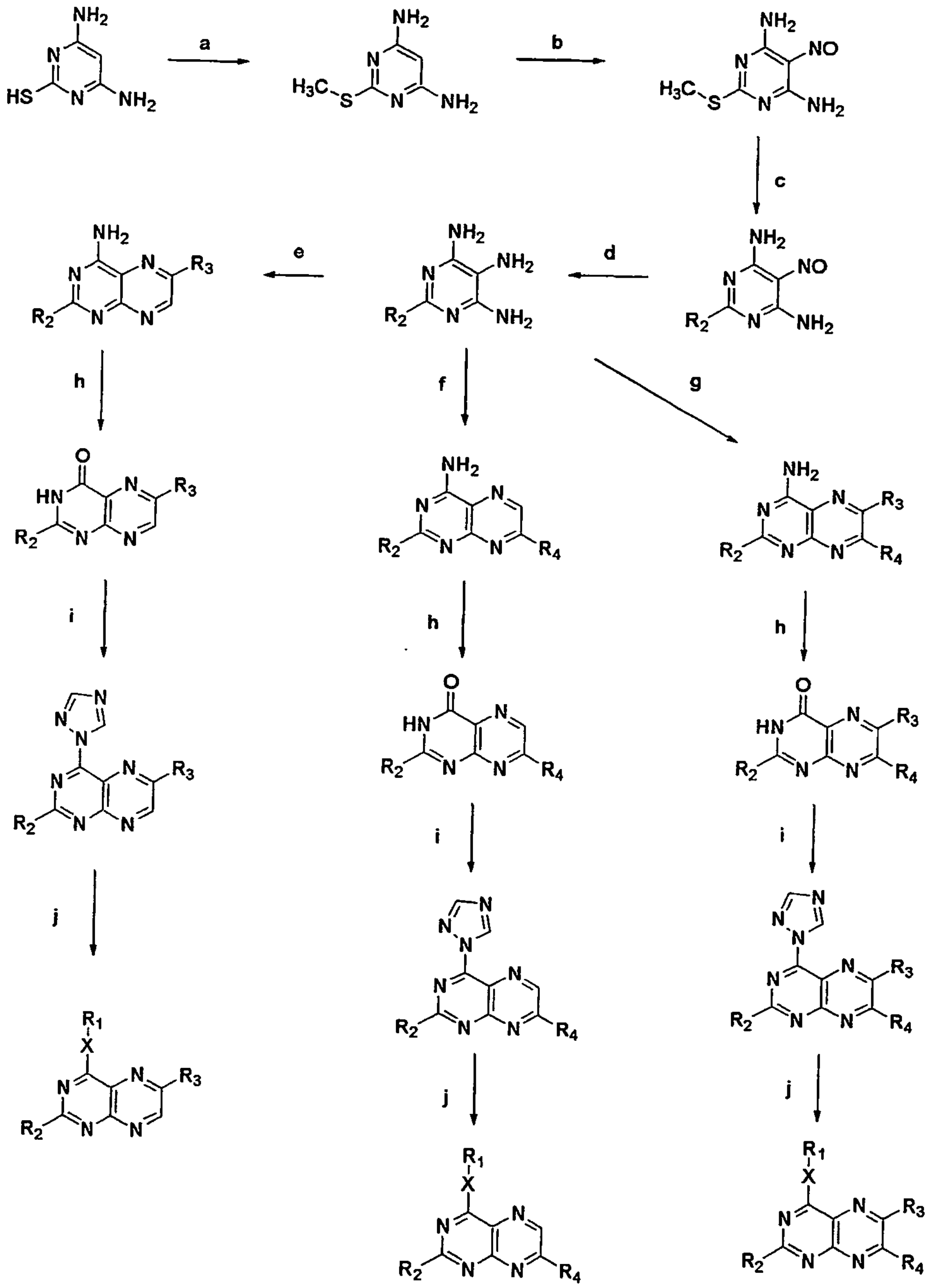

Figure 3

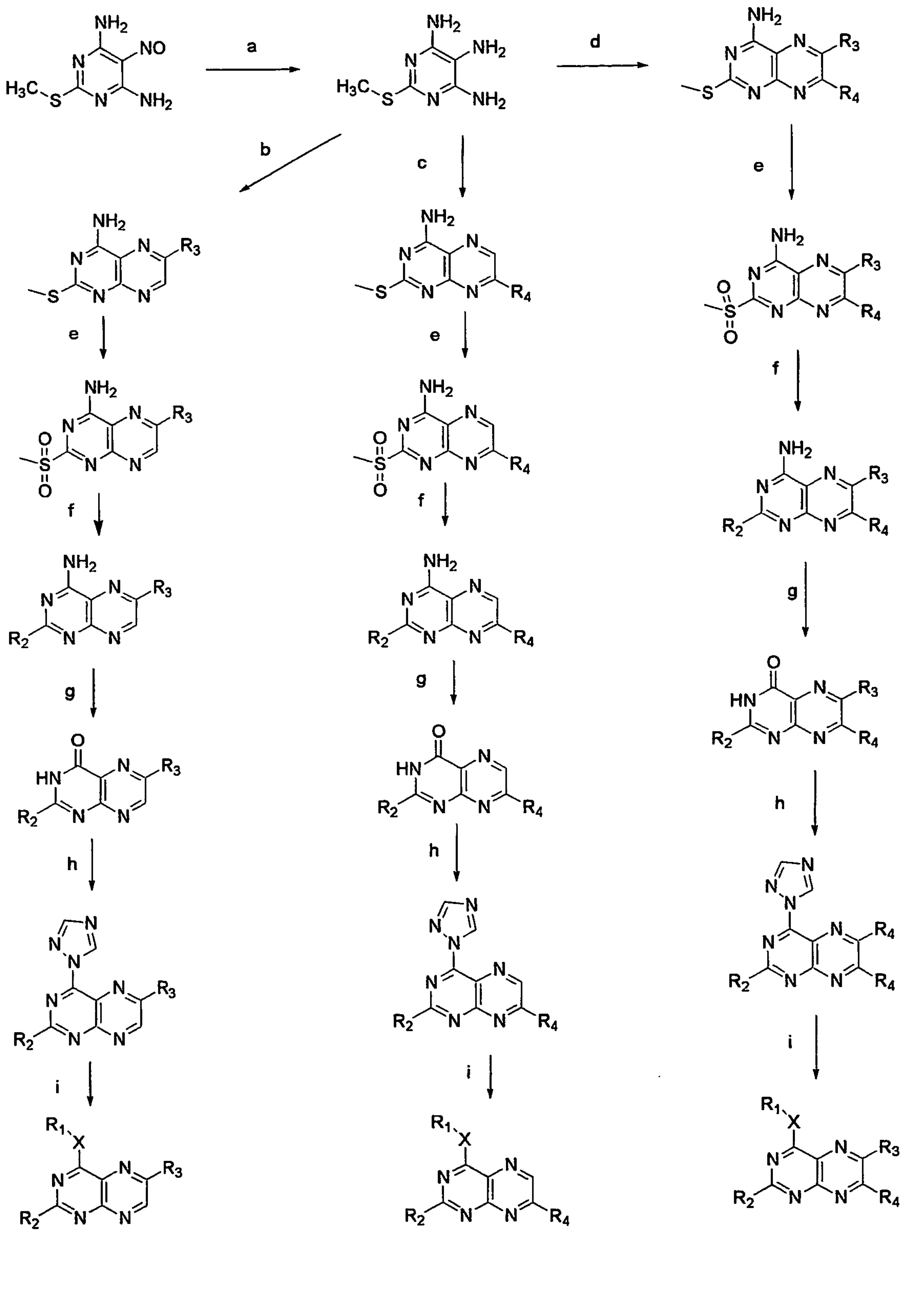

Figure 4

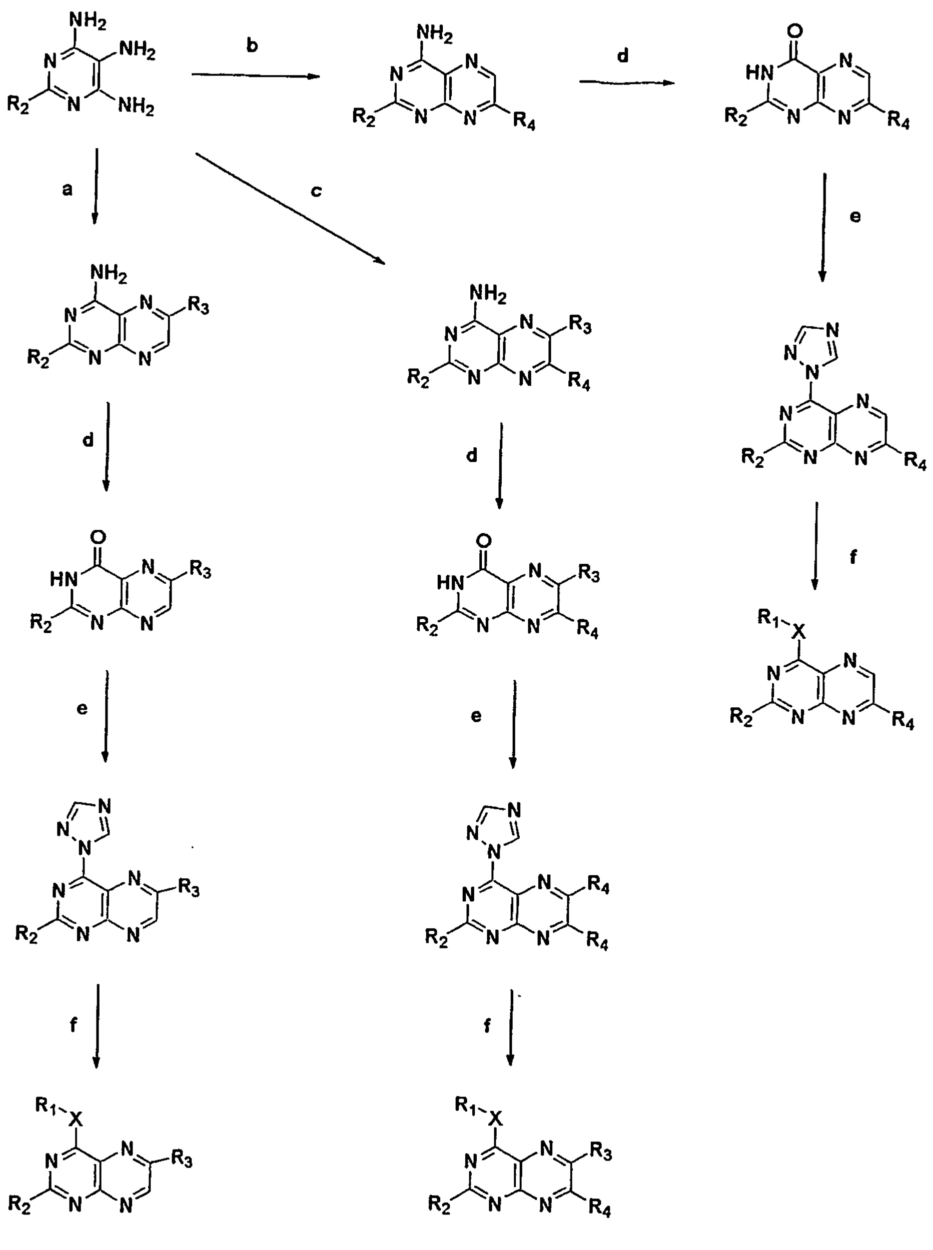

Figure 5

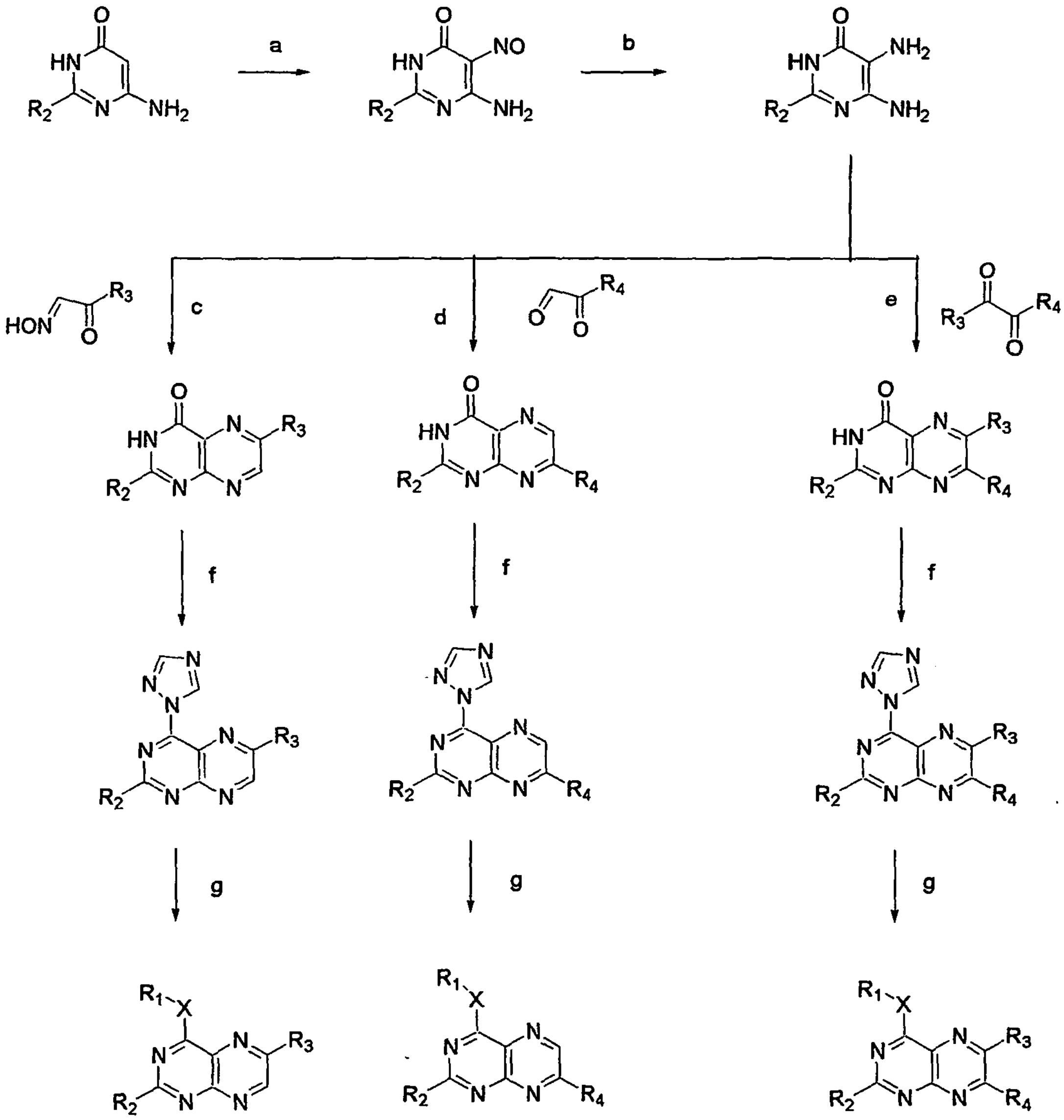

## Figure 6

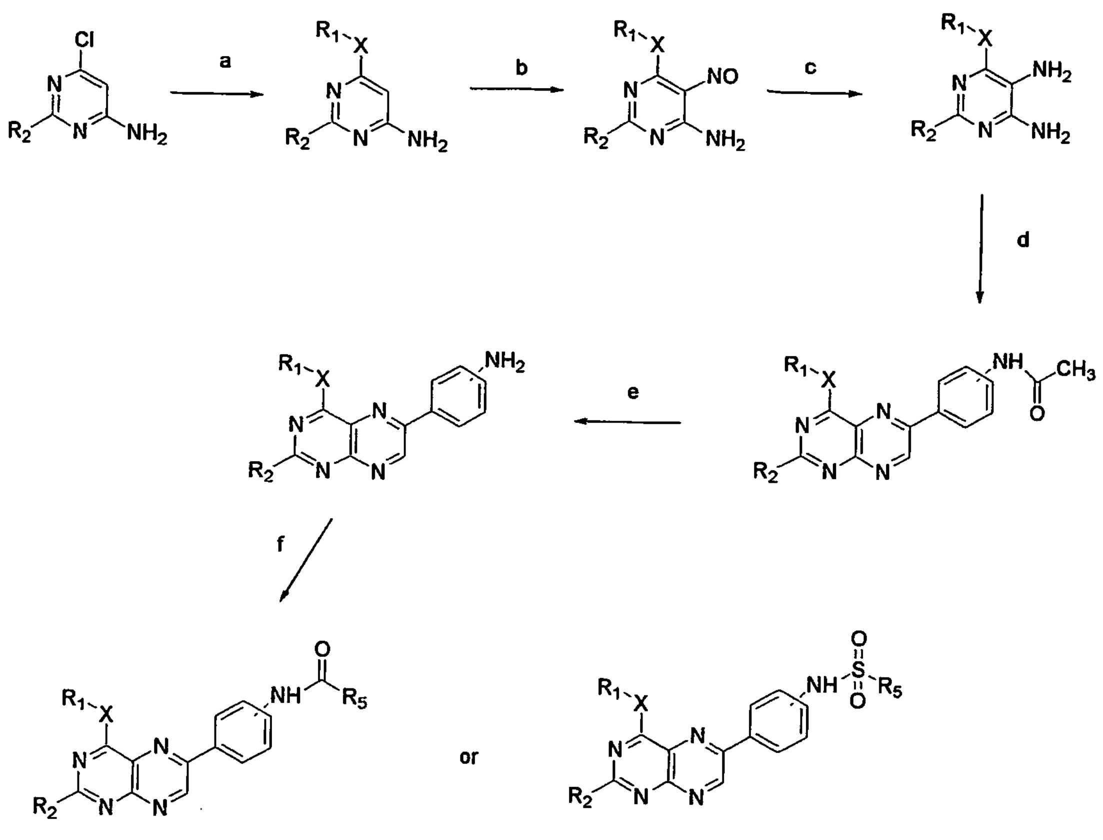


## Figure 7

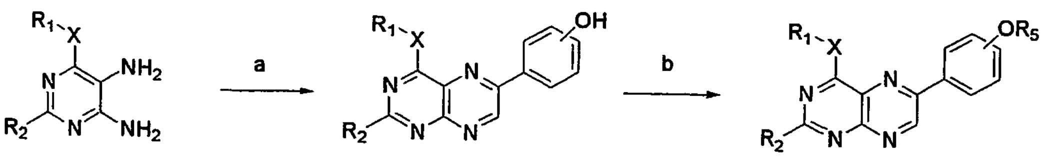

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CH 231852
- GB 763044 A
- US 2512572 A
- US 2581889 A
- US 2665275 A
- US 2667486 A
- US 2940972 A
- US 3081230 A
- US 5047405 A
- EP 362645 A
- US 2740784 A
- WO 0119825 A
- WO 0039129 A
- WO 0121619 A
- US 5665772 A
- US 5780462 A
- US 6440991 B
- US 6331547 B


### Non-patent literature cited in the description


- *J. Chem. Soc.,* 1957, 2146-2158
- *Helv. Chem. Acta,* 1992, vol. 75, 2317-2326
- **CHOU et al.** *Adv. Enzyme Reg.,* 1984, vol. 22, 27
- McCutcheon's Detergents and Emulsifiers Annual. 1981
- Tensid-Taschenbuch. Hanser Verlag, 1981
- Encyclopaedia of Surfactants. Chemical Publishing Co, 1981
- **TAYLOR et al.** *J. Am. Chem. Soc.,* 1952, vol. 74, 1644-1647